(19) Europäisches Patentamt
European Patent Office
Office européen des brevets

(11) EP 1 923 050 A1

(12) **EUROPEAN PATENT APPLICATION**

(43) Date of publication:
**21.05.2008 Bulletin 2008/21**

(51) Int Cl.:
***A61K 9/00*** *(2006.01)*

(21) Application number: **06291786.9**

(22) Date of filing: **17.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Besins Healthcare
1000 Bruxelles (BE)**

(72) Inventor: **Mesini-Eteve, Valérie
91370, Verrieres Le Buisson (FR)**

(74) Representative: **Nargolwalla, Cyra
Cabinet Plasseraud
52 rue de la Victoire
75440 Paris Cedex 09 (FR)**

(54) **Liquid pharmaceutical compositions comprising a bisphosphonate compound**

(57) The present invention relates to pharmaceutical compositions comprising a bisphosphonate compound.

EP 1 923 050 A1

## Description

FIELD OF THE INVENTION

The present invention relates to pharmaceutical compositions comprising a bisphosphonate compound. In some embodiments, the pharmaceutical composition is non film-forming and suitable for non-occlusive transdermal or transcutaneous delivery. In some embodiments, the pharmaceutical composition is a liquid pharmaceutical composition.

BACKGROUND

Bisphosphonate compounds are known in the art. For example, alendronic acid is known from US 4 705 651. This compound is useful for treating bone-related diseases, and is typically administered using an oral route (see, e.g., EP 998 292). The oral route involves a number of disadvantages, especially in terms of patient compliance. The oral administration must be severely controlled (time of administration, type of beverage to use, standing position required, etc.), in order to get full benefit from the treatment. This generally leads to patients discontinuing the treatment (reduced persistence), which has been associated with the occurrence of gastrointestinal adverse events. Thus, an efficient alternative mode of administration would be highly beneficial.
EP 1 475 095 A1 discloses percutaneous compositions of incadronate and alendronate salts. However, these compositions are formulated for administration through occlusive systems, such as patches, plasters or tapes, where a very high dose is provided in the composition and the delivery is driven by an occlusive membrane. Such systems usually involve the use of an adhesive, which may irritate skin, thus also potentially leading to treatment discontinuation. In addition, patches are non-aesthetically pleasing.
US 6 962 691 describes film-forming compositions for topical application of pharmaceutical compounds, including alendronate sodium. The compositions comprise film-forming acrylic polymers and/or copolymers which are said to form a breathable film on the surface of skin that is resistant to removal by rubbing for a period of time of from at least about 24 hours up to about 5 days after administration.
There remains a need, therefore, for non film-forming pharmaceutical compositions suitable for non-occlusive transdermal or transcutaneous delivery of bisphosphonates.

SUMMARY

The present invention relates to a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

The composition may be a stable, macroscopically homogenous solution.
The liquid pharmaceutical composition may consist essentially of a therapeutically effective amount of at least one bisphosphonate and water.
The liquid pharmaceutical composition may comprise an amount (w/w) of bisphosphonate in its free acid form selected from 0.05 - 7.5 %, 0.1-6%, 0.2-5%, 0.5-4.5%, 0.75-4%, 1-3%, or 1.5-2.5%, or an equivalent amount of bisphosphonate salt.
The liquid pharmaceutical composition may comprise alendronate at 90% saturation in pure water.
The liquid pharmaceutical composition may comprise anhydrous monosodium alendronate at 28.09 mg/g composition.
The liquid pharmaceutical composition may comprise an amount (w/w) of alendronate as a monosodium salt trihydrate selected from 0.05-3.8%, 0.1-3.75%, 0.5-3.75%, 0.75-3.75%, 1-3.75%, 1.5-3.75%, 2-3.75%, 2.5-3.75%,

3-3.75%, or 3.25-3.75%.

The liquid pharmaceutical composition may comprise risedronate at 90% saturation in pure water.

The liquid pharmaceutical composition may comprise anhydrous monosodium risedronate at 45.3 mg/g composition.

The liquid pharmaceutical composition may comprise an amount (w/w) of risedronate as a monosodium salt hemipentahydrate selected from 0.05-5.9%, 0.1-5.9%, 0.5-5.9%, 0.75-5.9%, 1-5.9%, 2-5.9%, 3-5.9%, 3.5-5.9%, 4-5.9%, 4.5-5.9%, 4.75-5.9%, 5-5.9%, or 5.5-5.9%.

The invention also concerns a unit dose package comprising a therapeutically effective amount for topical administration of the liquid pharmaceutical composition.

The invention also concerns a device comprising:

(A) a reservoir containing a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface, and

(B) a topical applicator.

The reservoir may contain a unit dose of a therapeutically effective amount of at least one bisphosphonate.

The applicator may be a metered dose applicator.

The metered dose applicator may be adapted to dispense, as each metered dose, a unit dose of a therapeutically effective amount of at least one bisphosphonate.

The applicator may comprise an applicator selected from the group consisting of a dropper, pipette, swab, brush, cloth, pad, sponge, and solid support.

The applicator may comprise an applicator selected from the group consisting of an aerosol or non-aerosol spray device.

The applicator may comprise an opening provided with a removable device for opening the opening.

The applicator may comprise an opening provided with a nozzle or valve.

The reservoir comprises a liquid pharmaceutical composition as described above.

The invention further proposes a method of administering a therapeutically effective amount of at least one bisphosphonate to a patient in need thereof, comprising topically administering to a surface of skin of the patient a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

The invention further proposes a method for treating a bone-related disorder, comprising topically administering to a surface of skin of a patient in need thereof an effective amount of a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

Said bone-related disorder may be selected from the group consisting of osteoporosis, menopause-associated osteoporosis, glucocorticoid-induced osteoporosis, Paget's disease, abnormal bone resorption, bone cancer, generalized bone loss, localized bone loss, bone metastasis with or without hypercalcemia, multiple myeloma and other conditions of bone fragility.

Said method may result in at least one therapeutic effect selected from the group consisting of reduced fracture frequency, increased bone (mineral) density, decreased alkaline phosphatase, osteocalcin, decreased N telopeptide collagen I, improved bone architecture, improved bone biomechanical properties (bone strength), for example as can be seen with bending, torsion and/or compression tests, and combinations thereof.

The administering may result in a ratio of urinary recovery after dermal administration versus intravenous administration of from 0.1- 5%.

The method may comprise administering a composition as described above.

The invention also concerna a use of a bisphosphonate in the manufacture of a medicament for treating and/or preventing a bone-related disorder, wherein said medicament is a composition as defined above.

BRIEF DESCRIPTION OF THE DRAWINGS

Figures 1-13 illustrate the absorption results obtained with compositions of the invention in an in vitro Franz cell assay.
Figure 1 shows the percentage of alendronate recovered in the receptor fluid and the dermis at 24 hrs using compositions with the water/alcohol ratios specified in the figure.
Figure 2 shows the percentage of risedronate recovered in the receptor fluid and the dermis at 24 hrs using compositions with the water/alcohol ratios specified in the figure.
Figure 3 shows the effect of the replacement of water by phosphate buffer pH 6 in compositions comprising 10% ethanol. (A = alendronate; R = risedronate)
Figure 4 shows a comparison of compositions comprising ethanol/water versus pure aqueous compositions, with the results reported in terms of the percent of administered dose.
Figure 5 shows a comparison of compositions comprising ethanol/water versus pure aqueous compositions, with the results reported in terms of amount.
Figure 6 shows the effect of menthol on percutaneous absorption of alendronate in buffered hydroalcoholic solution.
Figure 7 shows the effect of menthol on percutaneous absorption of risedronate in buffered hydroalcoholic solution.
Figure 8 shows the effect of urea on percutaneous absorption of alendronate in buffered hydroalcoholic solution.
Figure 9 shows the effect of urea on percutaneous absorption of risedronate in buffered hydroalcoholic solution.
Figure 10 shows the effect of urea and propylene glycol (PG) on percutaneous absorption of alendronate in buffered solution.
Figure 11 shows the effect of urea and propylene glycol (PG) on percutaneous absorption of risedronate in buffered solution.
Figure 12 shows the effect of oleic acid (OA) in the presence of Tween 80 (T80) and of glycerine on percutaneous absorption of alendronate in buffered hydroalcoholic solution.
Figure 13 shows the effect of oleic acid (OA) in the presence of Tween 80 (T80) and of glycerine on percutaneous absorption of risedronate in buffered hydroalcoholic solution.

DETAILED DESCRIPTION

In accordance with one aspect, the invention provides a pharmaceutical composition comprising bisphosphonate. In some embodiments, the composition is suitable for non-occlusive transdermal or transcutaneous delivery, such as for direct non-occlusive transdermal or transcutaneous delivery. In some embodiments, the composition is non-occlusive and/or non-film forming. In some embodiments, the composition is a liquid composition.

In one embodiment, the pharmaceutical composition comprises:

a therapeutically effective amount of at least one bisphosphonate,
optionally, a non-irritating amount of at least one moisturizer,
optionally, at least one short-chain aliphatic alcohol,
optionally, at least one gelling agent,
optionally, at least one surfactant, and
water.

As used herein "a" or "an" means one or more, unless specifically indicated to mean only one.

Unless otherwise stated, percentages (%) refer to amounts by weight based upon total weight of the composition (w/w).

In some embodiments, the composition is non-occlusive. As used herein, "non-occlusive" specifies that the composition is not provided in a patch, plaster, bandage, tape, or other form comprising a membrane, and that does not rely on a membrane to drive delivery of the pharmaceutical composition into the skin.

In some embodiments, the composition is non film-forming. As used herein, "non film-forming" specifies that the composition does not form a film on a skin surface that persists for a period of time of at least about 24 hours (such as at least 24 hours) after administration, e.g. the composition does not form a film that is resistant to removal by rubbing for such an extended period of time. In some embodiments, the non film-forming composition does not comprise an amount of a film-forming polymer, such as an acrylic film-forming polymer or co-polymer, sufficient to form a film on a skin surface that persists for a period of time of at least about 24 hours (such as at least 24 hours) after administration. As used herein "at least about 24 hours" includes, for example, at least 18 hours, at least 20 hours, at least 22 hours, and at least 24 hours.

In some embodiments, the composition is macroscopically homogenous. As used herein, "macroscopically homogenous" refers to the appearance of the composition upon visual inspection under typical conditions of use, such as room temperature, and specifies a composition that appears to comprise a single phase and does not appear to comprise macroscopically detectable crystals. For example, visual inspection of a macroscopically homogenous composition at room temperature indicates that the composition does not comprise crystals of one or more of the ingredients and does not reveal several phases that can be distinguished by simple visual inspection. Examples of macroscopically homogenous compositions include:

- a solution, wherein all ingredients are solubilized, i.e. all ingredients are below the saturation point;
- a macroscopically homogenous foam, such as a foam comprising foam pores with an average maximum diameter of about 200 μm, such as an average maximum diameter of 200 μm or an average maximum diameter of 200 μm +/- 20 μm;
- a macroscopically homogenous emulsion comprising droplets that are not distinguishable by simple visual inspection, such as an emulsion comprising droplets with an average maximum diameter of about 200 μm, such as an average maximum diameter of 200 μm or an average diameter of 200 μm +/-20 μm;
- a macroscopically homogenous gel or macroscopically homogenous cream or macroscopically homogenous ointment, such as a gel, cream or ointment that does not comprise clots detectable by touch.

In some embodiments, the macroscopically homogenous compositions do not include crystals and/or clots and/or solid agglomerates with an average maximum diameter larger than 200 μm.

As used herein, a macroscopically homogenous composition does not include suspensions comprising macroscopic crystals, such as crystals that are detectable with the naked eye, upon visual inspection.

Thus, the compositions of the present invention are distinguishable by physical properties from known bisphosphonate compositions. For example, while pharmaceutical formulations for occlusive systems may comprise suspensions wherein not all of the components are solubilized, the present invention provides macroscopically homogenous compositions.

The macroscopically homogenous compositions of the present invention are stable over time, in that, upon storage under standard storage conditions (e.g. room temperature), the macroscopically homogenous appearance is conserved. For example, over time, the macroscopically homogenous compositions do not exhibit phase separation or

demixing, and do not reveal crystallization of one or more of the ingredients (e.g., the property of no visible crystals is retained). In some embodiments, the compositions have a shelf life stability at room temperature of at least 2-3 months, at least 6 months, and/or at least 12 months. For practical purposes, a minimum stability requirement is the minimum time the composition is stored prior to packaging step, which may be a few hours (such as from 1-3 hours, from 3-8 hours, from 8-12 hours, etc.), one day, a few days (such as from 1-3 days, from 3-5 days, from 5-7 days, etc.), one week, a few weeks (such as from 1-3 weeks, from 3-5 weeks, etc.), one month, a few months (such as from 1-3 months, from 3-5 months, from 5-7 months, from 7-9 months, from 9-12 months, etc.), or one year or longer. The skilled person can readily determine if such a stability requirement is met. For example, the skilled person can use standard solubility studies to determine appropriate solubility parameters. Thus, in one embodiment, the macroscopically homogenous compositions of the present invention are stable over a period of time of few hours (such as from 1-3 hours, from 3-8 hours, from 8-12 hours, etc.), one day, a few days (such as from 1-3 days, from 3-5 days, from 5-7 days, etc.), one week, a few weeks (such as from 1-3 weeks, from 3-5 weeks, etc.), one month, a few months (such as from 1-3 months, from 3-5 months, from 5-7 months, from 7-9 months, from 9-12 months, etc.), or one year or longer.

In some embodiments, the compositions according to the invention do not require any adhesive for administration. Such embodiments offer clear advantages over known compositions that require an adhesive, such as avoiding the use of potentially irritating ingredients.

The compositions of the invention offer further advantages, including being non-irritating to the skin and resulting in limited side effects. As a result of these and other advantages, the compositions facilitate patient compliance.

## Compositions

As noted above, a pharmaceutical composition of the present invention may comprise

a therapeutically effective amount of at least one bisphosphonate,
optionally, a non-irritating amount of at least one moisturizer,
optionally, at least one short-chain aliphatic alcohol,
optionally, at least one gelling agent,
optionally, at least one surfactant, and
water.

In one embodiment, the pharmaceutical composition is a liquid pharmaceutical composition. In one embodiment, the pharmaceutical composition does not comprise a gelling agent. In one embodiment, the pharmaceutical composition does not comprise a moisturizer. In one embodiment, the pharmaceutical composition does not comprise a short-chain aliphatic alcohol. In one embodiment, the pharmaceutical composition does not comprise a surfactant.

Thus in one embodiment, a pharmaceutical composition of the present invention comprises a therapeutically effective amount of at least one bisphosphonate and water. In another embodiment, a pharmaceutical composition of the present invention consists of a therapeutically effective amount of at least one bisphosphonate and water.

In yet another embodiment, a pharmaceutical composition of the present invention consists essentially of a therapeutically effective amount of at least one bisphosphonate and water. As used herein, "consists essentially of at least one bisphosphonate and water" means that the composition includes at least one bisphosphonate and water, and may also include other components that do not materially affect the basic and novel characteristics of the invention, such as, for example, its stability, its macroscopic homogeneity, its non-occlusive nature and its non film-forming nature. Thus, for example, a composition consisting essentially of a therapeutically effective amount of at least one bisphosphonate and water may include another bisphosphonate. On the other hand, a composition consisting essentially of a therapeutically effective amount of at least one bisphosphonate and water will not include an amount of a film-forming polymer, such as an acrylic film-forming polymer or co-polymer, sufficient to form a film on a skin surface that persists for a period of time of at least about 24 hours (such as at least 24 hours) after administration.

In some embodiments, the components are provided in the form of a stable, macroscopically homogenous mixture, as discussed above. In some embodiments, the compositions are non film-forming and/or non-occlusive.

In some embodiments, the composition of the invention has a pH of between about 4.0 and about 8.5, such as a pH is in the ranges 4.0-8.5, 4.5-8.0, 5.0-7.5, 5.5-7.0, 5.0-6.0, 6.0-7.0 or 6.5-7.5. Such pH values readily can be reached with buffering compounds. Useful buffering compounds are known in the art, and include phosphate and citrate buffers, including sodium citrate, or tris maleate. Those skilled in the art can select suitable buffering agents, and appropriate concentrations to achieve the desired pH.

As noted above, compositions of the invention are suitable for topical administration. For example, the compositions can be directly applied to a surface of the skin, for direct non-occlusive transdermal/transcutaneous application. As used herein, the terms "direct"/"directly" and "non-occlusive" reflect that the compositions of the invention do not require a matrix or membrane to effect administration, and thus are not required to be dispensed via a patch, plaster,

tape system, or the like. Moreover, the compositions of the invention do not require an adhesive for administration. Instead, the compositions of the invention are formulated for delivery of bisphosphonate by direct application of the composition onto a surface of the skin. In some embodiments, the amount of composition administered is a defined, finite amount that provides a therapeutically effective amount (e.g., a single dose) of bisphosphonate. As described in more detail below, a "therapeutically effective amount" specifies an amount sufficient to achieve an intended therapeutic effect in a given patient (e.g., a human or other animal). In some embodiments, the composition is administered to a surface of the skin over a defined surface area. The administration of a defined, finite amount of the composition to a defined surface area permits the control of the amount of active principle (e.g., bisphosphonate) that is applied to a given surface area, e.g., the local concentration. By controlling (e.g., limiting) local concentration, skin irritation that may be caused by the composition can be reduced, and side effects, such as GI tract irritation, can be minimized. In the context of the present invention, the ability to control local concentration is not limited by the size or dimensions of a membrane or occlusive structure, such as a patch. Thus, the composition can be administered over a larger surface area than might be possible, feasible or aesthetically acceptable with an occlusive device.

For example, the composition can be applied onto a surface of the skin with a surface area of from about 1000 $cm^2$ (e.g., the approximate area of about half a forearm of an adult, human patient) to about 4000 $cm^2$ (e.g., the approximate area of two arms, or the approximate area of two upper arms plus the abdomen, of an adult, human patient), or larger. For example, a surface area of about 1000 $cm^2$ is suitable for the application of up to about 2 g of the composition, while a surface area of about 4000 $cm^2$ is suitable for the application of up to about 10-12 g of the composition. As used herein, "a surface area of from about 1000 $cm^2$" includes a surface area of 1000 $cm^2$ +/- 200 $cm^2$ and larger. As used herein, "a surface area of from about 4000 $cm^2$" includes a surface area of 4000 $cm^2$ +/- 800 $cm^2$ and larger. Those skilled in the art will readily be able to determine appropriate surface areas for the topical application of a given amount of composition to a given patient.

## Bisphosphonates

As noted above, the compositions of the invention comprise a therapeutically effective amount of at least one bisphosphonate.

As used herein, "bisphosphonate" includes a bisphosphonic acid in its free acid form, any of its pharmacologically acceptable salts, any of its pharmacologically acceptable esters, any hydrate thereof, any derivative thereof bearing one or two methyl group(s) on the amino function, and mixtures of one or more of the foregoing. The counter-ion for a bisphosphonic salt may be any pharmaceutically suitable counter-ion, such as any pharmaceutically suitable cation. For example, the counter-ion can be sodium, potassium, magnesium, or calcium, a small amine moiety, such as lysine or a small poly-lysine. A bisphosphonic ester can be a mono-, di-, tri- or tetra-ester of bisphosphonic acid, esterified at one or more of the four acidic hydroxyl groups of the bisphosphonic acid. In some embodiments, the esters are C1-C3 esters, such as methyl or ethyl esters. In some embodiments, each hydroxyl group is modified by the same alcohol, but other embodiments include so-called 'mixed' esters, wherein the bisphosphonic acid is esterified with two or more different alcohols.

In one aspect of the invention, the bisphosphonate has the structure of formula I

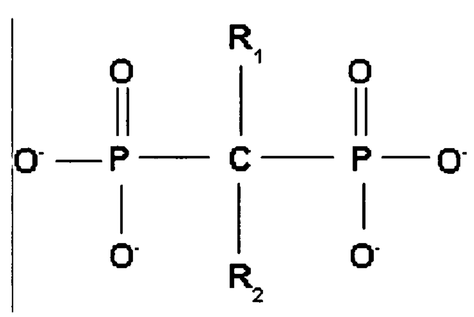

(formula I)

wherein:

- R1 is H, OH or Cl; and
- R2 is:

  ■ alkyl with 1, 2, 3, 4, 5, or 6 carbon atoms, optionally substituted with amino, alkylamino, dialkylamino or heterocyclyl, e.g. N-heterocyclyl or N,N'-heterocyclyl;

- ■ halogen (F, Cl, Br, I);
- ■ arylthio, including chlorosubstituted arylthio;
- ■ cycloalkylamino with 5, 6 or 7 carbons; or
- ■ saturated five or six-membered nitrogen-containing heterocyclyl with 1 or 2 heteroatoms.

Alkyl groups in the above alkylamino and dialkylamino groups may have 1, 2, 3, 4, or 5 carbon atoms. The dialkylamino groups may comprise the same or different alkyl groups, e.g., each alkyl group of a dialkylamino group is selected independently.

In the above formula, the term "heterocyclyl" means a saturated or unsaturated 5-, 6-, or 7-membered heterocyclic group with one or two rings and 1, 2, or 3 heteroatoms, independently chosen from N, O and S.

In the above formula, the term "aryl" denotes a substituted or unsubstituted phenyl, furyl, thienyl or pyridyl group, or a fused ring system of any of these groups, such as naphtyl.

In the above formula, the term "substituted" denotes an aryl group as defined above which is substituted by one or more alkyl (e.g. C1-C6 alkyl, linear or branched), alkoxy (e.g. C1-C6 alkoxy, linear or branched), halogen (F, Cl, Br, I), amino, thiol, nitro, hydroxy, acyl, aryl or cyano groups.

Examples of bisphosphonates useful in the present invention include compounds of formula I, wherein R1 and R2 have the following definitions:

| Agent | $R_1$ side chain | $R_2$ side chain |
|---|---|---|
| Etidronate | -OH | $-CH_3$ |
| Clodronate | -Cl | -Cl |
| Tiludronate | -H | 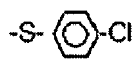 |
| Pamidronate | -OH | $-CH_2-CH_2-NH_2$ |
| Neridronate | -OH | $-(CH_2)_5-NH_2$ |
| Olpadronate | -OH | $-(CH_2)_2(CH_3)_2$ |
| Alendronate | -OH | $-(CH_2)_3-NH_2$ |
| Ibandronate | -OH | 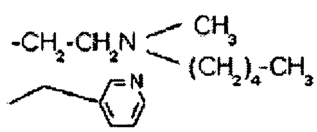 |
| Risedronate | -OH | |
| Zoledronate | -OH | 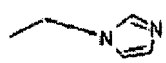 |

In one aspect, R1 is -OH, and R2 is selected from alkyl groups with 1, 2, 3, 4, 5, or 6 carbon atoms, optionally substituted with amino, alkylamino, dialkylamino or heterocyclyl, e.g. N-heterocyclyl or N,N'-heterocyclyl.

In another aspect, the bisphosphonate is selected form the group consisiting of:

- 4-amino-1-hydroxybutylidene-1,1-bisphosphonic acid (alendronate),
- N,N-dimethyl-3-amino-1-hydroxypropylidene-1,1-bisphosphonic acid (mildronate, olpadronate),
- 1-hydroxy-3-(N-methyl-N-pentylamino)propylidene-1,1-bisphosphonic acid (ibandronate),
- 1-hydroxy-2-(3-pyridyl)ethylidene-1,1-bisphosphonic acid (risedronate),
- 1-hydroxyethylidene- 1,1-bisphosphonic acid (etidronate),
- 1-hydroxy-3-(1-pyrrolidinyl)propylidene-1,1-bisphosphonic acid,
- 1-hydroxy-2-(1-imidazolyl)etylidene-1,1-bisphosphonic acid (zoledronate),
- 1-hydroxy-2-(imidazo[1,2-a]pyridin-3-yl)ethylidene-1,1-bisphosphonic acid (minodronate),
- 1-(4-chlorophenylthio)methylidene-1,1-bisphosphonic acid (tiludronate),
- 1-(cycloheptylamino)methylidene-1,1-bisphosphonic acid (cimadronate, incadronate),
- 6-amino-1-hydroxyhexylidene-1,1-bisphosphonic acid (neridronate)
- (dichloromethylene)-bisphosphonic acid (Clodronate, Bonefos®, Loron®)
- (3-amino-1-hydroxypropylidene)-bisphosphonic acid (Pamidronate, APD, Aredia®)
- [1-hydroxy-2-(imidazo[1,2-a]pyridin-3-yl)ethylidene]-bisphosphonic acid (minodronate).

In one aspect, the bisphosphonate is selected from the group consisting of alendronate and risedronate. In another aspect, the bisphosphonate is not incadronate. As used herein, "alendronate" includes alendronic acid (4-amino-1-hydroxybutylidene-1,1-bisphosphonic acid) in its free acid form, any of its pharmacologically acceptable salts, any of its pharmacologically acceptable esters, any hydrate thereof, any derivative thereof bearing one or two methyl group(s) on the amino function, and mixtures of any one or more of the foregoing. The counter-ion for an alendronate salt may be any pharmaceutically suitable counter-ion, such as any pharmaceutically suitable cation. For example, the counter-ion can be sodium, potassium, magnesium, or calcium, or may be a small amine moiety, such as lysine or a small poly-lysine. An alendronate ester can be a mono-, di-, tri- or tetra-ester of alendronic acid, esterified at one or more of the four acidic hydroxyl groups of alendronate. In some embodiments, the esters are C1-C3 esters, such as methyl and ethyl esters. In some embodiments, all hydroxyl groups are modified by the same alcohol, but other embodiments include so-called ’mixed’ esters, wherein the alendronate is esterified with two or more different alcohols.

As used herein "risedronate" and "residronate" specify a risedronic acid (residronic acid or 1-hydroxy-2-(3-pyridyl)ethylidene-1,1-bisphosphonic acid) in its free acid form, any of its pharmacologically acceptable salts, any of its pharmacologically acceptable esters, any hydrate thereof, any derivative thereof bearing one or two methyl group(s) on the amino function, and mixtures of any one or more of the foregoing. A counter-ion for a risedronic salt may be any pharmaceutically suitable counter-ion, such as any pharmaceutically suitable cation. For example, the counter-ion can be sodium, potassium, magnesium, or calcium, or may be a small amine moiety, such as lysine or a small poly-lysine. A risedronate ester can be a mono-, di-, tri- or tetra-esters of risedronic acid, esterified at one or more of the four acidic hydroxyl groups of the risedronic acid. In some embodiments, the esters are C1-C3 esters, such as methyl and ethyl esters. In some embodiments, all hydroxyl groups are modified by the same alcohol, but other embodiments include so-called ’mixed’ esters, wherein the risedronic acid is esterified with two or more different alcohols.

In some embodiments, the pharmaceutical compositions of the invention comprise at least one further active ingredient, e.g., another bisphosphonate compound, such as may be desired for combination therapy.

As noted above, the composition comprises a therapeutically effective amount of at least one bisphosphonate. A therapeutically effective amount generally depends on the potency of the bisphosphonate, its molecular weight, and other factors. The skilled person knows from available literature appropriate ranges of amounts of the above-described bisphosphonates, or can readily determine therapeutically effective amounts using routine methods. Information on the bioavailability of bisphosphonates administered in accordance with the present invention is provided below. Also provided below are alternative models for determining appropriate amounts for dermal delivery based on oral dosages. Those skilled in the art can use these or other methods to determine a therapeutically effective amount of bisphosphonate for use in accordance with the invention.

## Moisturizers

As noted above, the compositions of the invention may optionally comprise a non-irritating amount of at least one moisturizer.

As used herein "moisturizer" specifies an agent that hydrates the skin. Moisturizers are known in the art. Moisturizers can be used either alone or in combination, e.g., a combination of two or three (or more) different moisturizers can be used. In some embodiments, moisturizers are selected from emollients and/or humectants.

As used herein, "emollients" specify substances that soften the skin and tend to improve moisturization of the skin. Emollients are well known in the art, and include mineral oil, petrolatum, polydecene, isohexadecane, fatty acids and alcohols having from 10 to 30 carbon atoms; pelargonic, lauric, myristic, palmitic, stearic, isostearic, hydroxystearic, oleic, linoleic, ricinoleic, arachidic, behenic, and euricic acids and alcohols; triglyceride esters, castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, acetoglyceride esters, ethoxylated glycerides, ethoxylated glyceryl monostearate, alkyl esters of fatty acids having 10 to 20 carbon atoms, hexyl laurate, isohexyl laurate, isohexyl palmitate, isopropyl palmitate, decyl oleate, isodecyl oleate, hexadecyl stearate, decyl stearate, diisopropyl adipate, diisohexyl adipate, diisopropyl sebacate, laurly lactate, myristyl lactate, acetyl lactate; alkenyl esters of fatty acids having 10 to 20 carbon atoms, oleyl myristate, oleyl stearate, oleyl oleate, fatty acid esters of ethoxylated fatty alcohols, polyhydric alcohol esters, ethylene glycol mono and di-fatty acid esters, diethylene glycol mono- and di-fatty acid esters, polyethylene glycol, wax esters, beeswax, spermaceti, myristyl myristate, stearyl stearate, silicone oils, dimethicones, cyclomethicones. In some embodiments, the composition comprises one or more emollients that are liquid at room temperature.

In some embodiments, the composition further comprises a surfactant, which may help maintain the macroscopically homogenous property of the composition, which could be detrimentally affected by certain emollients. The skilled person can select suitable surfactant(s), and incorporate them in the composition in order to maintain macroscopic homogeneity.

As used herein "humectants" specifies hygroscopic substances that absorb water from the air. Humectants suitable for use in the invention include glycerine, propylene glycol, glyceryl triacetate, a polyol, sorbitol, maltitol, a polymeric polyol, polydextrose, quillaia, lactic acid, and urea.

Moisturizers suitable for use in the present invention may comprise amines, alcohols, glycols, amides, sulfoxides, and pyrrolidones. In one aspect, the moisturizer is selected from the group consisting of lactic acid, glycerine, propylene glycol, and urea.

As noted above, the compositions of the invention comprise an amount of moisturizer which is generally considered to be non-irritating to human skin, as determined by methods known in the art. For example, when using urea as a moisturizer, the amount thereof should not exceed the amount which is dermatologically acceptable. This is generally understood to mean that the concentration of urea should remain below 5% (w/w), or below 4% (w/w), in the compositions of the invention. Using common general knowledge, the skilled person can determine non-irritating amounts of moisturizer. In some embodiments, the non-irritating amount results in no detectable or sustained dermal adverse reaction (e.g., itching, reddening, burning sensation), or results in only a minimal reaction that is generally deemed to be acceptable by patients and health care providers.

### Short-Chain Aliphatic Alcohols

As noted above, the compositions of the invention may optionally comprise at least one short-chain aliphatic alcohol.

Exemplary short-chain aliphatic alcohols include C2-C4 alcohols, such as ethanol, n-propanol, isopropanol, n-butanol, tert-butanol, or mixtures thereof. The presence of such an alcohol may contribute to accelerated drying of the composition onto the skin.

### Gelling Agents

As noted above, the compositions of the invention may optionally comprise at least one gelling agent.

As used herein, the term "gelling agent" specifies a compound, optionally of polymeric nature, having the capacity to form a gel when contacted with a specific solvent, e.g., water. Gelling agents (e.g., thickeners) are known in the art. Gelling agents may act to increase the viscosity of the pharmaceutical compositions of the invention. For example, a gelling agent may provide the composition with sufficient viscosity to allow easy application of the composition onto the skin. Additionally or alternatively, gelling agents may act as solubilizing agents.

Examples of gelling agents include anionic polymers such as acrylic acid based polymers (including polyacrylic acid polymers, e.g. CARBOPOL® by Noveon, Ohio), cellulose derivatives, poloxamers and poloxamines, more precisely, Carbomers or acrylic acid-based polymers, e.g. Carbopol® 980 or 940, 981 or 941, 1342 or 1382, 5984, 934 or 934P (Carbopol® are usually polymers of acrylic acid crosslinked with allyl sucrose or allylpentaerythritol), Ultrez, Pemulen TR1® or TR2®, Synthalen CR, etc.; cellulose derivatives such as carboxymethylcelluloses, hydroxypropylcelluloses, hydroxyethylcelluloses, ethylcelluloses, hydroxymethylcelluloses, hydroxypropylmethylcelluloses, and the like, and mixtures thereof; poloxamers or polyethylene-polypropylene copolymers such as Lutrol ® grade 68 or 127, poloxamines and other gelling agents such as chitosan, dextran, pectins, and natural gums. Any one or more of these gelling agents may be used alone or in combination in the pharmaceutical compositions according to the invention. In one aspect, the gelling agent is selected from the group consisting of polyacrylic acids, cellulosics, and mixtures thereof.

### Surfactants

As noted above, the compositions of the invention may optionally comprise at least one surfactant.

Depending on the nature of the selected ingredients, it may be advantageous to include a surfactant, for example, to maintain the macroscopic homogeneity of the composition. Surfactants are known in the art, and the skilled person can select suitable surfactants in use for the present invention, such as surfactants that are dermatologically and/or cosmetically acceptable. Examples thereof include non-ionic surfactants, for example:

- esters, such as:

    o esters of polyethyleneglycol and fatty acids, including Labrasol®, which is a mixture of mono, di and triglycerides and of mono and diesters of polyethyleneglycol and fatty acids;
    o esters of saccharose and fatty acids, such as: sucrose laurate with HLB16; sucrose palmitate with HLB 16;
    o esters of sorbitanne polyoxyethylene, such as Tween® compounds including Tween® 20, 60 and/or 80;

- alkylene oxide copolymers, such as copolymers of ethylene oxide and propylene oxide, e.g. Pluronics®;

Further examples include anionic surfactants such as SDS (sodium dodecyl sulphate) and the like.

**Water**

As noted above, the composition of the invention comprises water.

**Further Optional Components**

The pharmaceutical compositions of the invention optionally may comprise other usual pharmaceutical additives, including salt(s), stabilizer(s), antimicrobial(s) such as paraben compounds, fragrance(s), and/or propellant(s). In one aspect, the compositions of the invention do not comprise menthol.

As noted above, in some embodiments, a non film-forming composition according to the invention does not comprise an amount of a film-forming polymer, such as an acrylic film-forming polymer or co-polymer, sufficient to form a film on a skin surface that persists for a period of time of at least about 24 hours (such as at least 24 hours) after administration.

**Exemplary Compositions**

In one aspect, the composition of the invention comprises 0.05 - 7.5 %, 0.1-6%, 0.2-5%, 0.5-4.5%, 0.75-4%, 1-3%, or 1.5-2.5%, of at least one bisphosphonate in its free acid form (free acid equivalent), or an equivalent amount of salt. The skilled person can compute equivalent amounts, e.g. if the bisphosphonate is provided as a salt with a counter ion.

In another aspect, the composition of the invention comprises alendronate as a monosodium salt. In one aspect, the composition comprises 0.05-3.8%, 0.1-3.75%, 0.5-3.75%, 0.75-3.75%, 1-3.75%, 1.5-3.75%, 2-3.75%, 2.5-3.75%, 3-3.75%, or 3.25-3.75%, of alendronate as a monosodium salt trihydrate.

In one embodiment, the composition comprises alendronate at 90% saturation in pure water, such as anhydrous monosodium alendronate at 28.09 mg/g composition.

In another aspect, the composition of the invention comprises risedronate as a monosodium salt. In one aspect, the composition of the invention comprises 0.05-5.9%, 0.1-5.9%, 0.5-5.9%, 0.75-5.9%, 1-5.9%, 2-5.9%, 3-5.9%, 3.5-5.9%, 4-5.9%, 4.5-5.9%, 4.75-5.9%, 5-5.9%, or 5.5-5.9%, of risedronate as a monosodium salt hemipentahydrate.

In one embodiment, the composition comprises risedronate at 90% saturation in pure water, such as anhydrous monosodium risedronate at 45.3 mg/g composition.

In one embodiment, the composition of the invention comprises alendronate as a monosodium salt trihydrate, at a concentration of 0.5-3.8% in a phosphate buffer. In another embodiment, the composition of the invention comprises risedronate as a monosodium salt hemipentahydrate, at a concentration of 0.5-5.9% in phosphate buffer.

In one aspect, the composition of the invention comprises 0.05 - 12 % of at least one moisturizer. As explained above, the moisturizer is present in a non-irritating amount. The composition of the invention may comprise 0.05-12%, 0.1-10%, 0.25-8%, 0.5-7%, 0.75-6%, 1-5%, or 1.5-4% of at least one moisturizer.

The composition of the invention may comprise urea as a moisturizer. Generally, a non-irritating amount of urea may correspond to 0.05-4%, 0.1-3.9%, 0.25-3.8%, 0.5-3.75%, 0.75-3.75%, 1-3.75%, 1.25-3.75%, 1.5-3.75%, 2-3.75%, or 2.5-3.5%, of urea. The composition of the invention may comprise glycerine as a moisturizer. Generally, a non-irritating amount of glycerine may correspond to 0.05-20%, 2-18%, 5-15%, 7-12%, 8-11%, 9-10%, or 10%, of glycerine.

The composition of the invention may comprise propylene glycol as a moisturizer. Generally, a non-irritating amount of propylene glycol may correspond to 0.05-12%, 1-11%, 2-10%, 3-10%, 4-10%, 5-9%, 6-9%, 7-9%, or 8-9%, of propylene glycol.

In another aspect, the pharmaceutical composition according to the invention may comprise 0-12%, 0.05-10%, 0.1-8%, 0.25-7%, 0.5-5%, 1-4%, or 2-3% of at least one short-chain aliphatic alcohol, e.g. ethanol.

The composition of the invention may comprise a gelling agent. In one aspect, the composition of the invention comprises 0-5 % of at least one gelling agent.

In another aspect, the pharmaceutical composition according to the invention comprises 0.02-5%, 0.05-5.0 %, 0.15-4.5 %, 0.2-4.0 %, 0.25-3.5 %, 0.3-3.0 %, 0.4-2.5 %, 0.5-2.0 %, or 0.3-1.5 %, of at least one gelling agent.

In another aspect, the pharmaceutical composition according to the invention comprises 0.5-10% of at least one surfactant. In another aspect, the composition of the invention comprises 0.02-5%, 0.05-5.0 %, 0.15-4.5 %, 0.2-4.0 %, 0.25-3.5 %, 0.3-3.0 %, 0.4-2.5 %, 0.5-2.0 %, or 0.3-1.5 %, of at least one surfactant.

In another aspect, the present invention relates to a pharmaceutical composition comprising (w/w):

- 0.05 - 7.5 % of at least one bisphosphonate,
- 0.05 - 12 % of at least one moisturizer,
- 0 - 12 % of at least one short-chain aliphatic alcohol,

- 0 - 5 % of at least one gelling agent,
- 0 - 10 % of a surfactant,
- 0-2.5 % buffer, and
- q.s. water,

In another aspect, the present invention relates to a pharmaceutical composition comprising (w/w):

- 0.05-3.8% of alendronate as a monosodium salt trihydrate,
- 0.05 - 12 % of at least one moisturizer,
- 0 - 12 % of at least one short-chain aliphatic alcohol,
- 0 - 5 % of at least one gelling agent,
- 0 - 10 % of a surfactant,
- 0-2.5 % buffer, and
- q.s. water.

In another aspect, the present invention relates to a pharmaceutical composition comprising (w/w):

- 0.05-3.8% of alendronate as a monosodium salt trihydrate,
- 0.05-4% of urea; or 0.05-20% of glycerine; or 0.05-12% of propylene glycol;
- 0 - 12 % of ethanol,
- 0-5% of at least one gelling agent,
- 0-10% of a surfactant,
- 0-2.5 % buffer, and
- q.s. water.

In another aspect, the present invention relates to a pharmaceutical composition comprising (w/w):

- 1-3.75% of alendronate as a monosodium salt trihydrate,
- 1-3.75% of urea; or 5-15% of glycerine; or 4-10% of propylene glycol;
- 0 - 12 % of ethanol,
- 0 - 5 % of at least one gelling agent,
- 0 - 10 % of a surfactant,
- 0-2.5 % buffer, and
- q.s. water.

In another aspect, the present invention relates to a pharmaceutical composition comprising (w/w):

- 3.25-3.75% of alendronate as a monosodium salt trihydrate.
- 2.5-3.5% of urea; or 7-12% of glycerine; or 8-9% of propylene glycol
- 0 - 12 % of ethanol,
- 0 - 5 % of at least one gelling agent,
- 0 - 10 % of a surfactant,
- 0-2.5 % buffer, and
- q.s. water.

In another aspect, the present invention relates to a pharmaceutical composition comprising (w/w):

- 0.05-5.9% of risedronate as a monosodium salt hemipentahydrate,
- 0.05 - 12 % of at least one moisturizer,
- 0 - 12 % of at least one short-chain aliphatic alcohol,
- 0 - 5 % of at least one gelling agent,
- 0 - 10 % of a surfactant,
- 0-2.5 % buffer, and
- q.s. water.

In another aspect, the present invention relates to a pharmaceutical composition comprising (w/w):

- 0.05-5.9% of risedronate as a monosodium salt hemipentahydrate,

- 0.05-4% of urea; or 0.05-20% of glycerine; or 0.05-12% of propylene glycol;
- 0 - 12 % of ethanol,
- 0 - 5 % of at least one gelling agent,
- 0 - 10 % of a surfactant,
- 0-2.5 % buffer, and
- q.s. water.

In another aspect, the present invention relates to a pharmaceutical composition comprising (w/w):

- 2-5.9% of risedronate as a monosodium salt hemipentahydrate
- 1-3.75% of urea; or 5-15% of glycerine; or 4-10% of propylene glycol;
- 0 - 12% of ethanol,
- 0 - 5% of at least one gelling agent,
- 0 - 10% of a surfactant,
- 0-2.5% buffer, and
- q.s. water.

In another aspect, the present invention relates to a pharmaceutical composition comprising (w/w):

- 5.5-5.9% of risedronate as a monosodium salt hemipentahydrate.
- 2.5-3.5% of urea; or 7-12% of glycerine; or 8-9% of propylene glycol
- 0 - 12 % of ethanol,
- 0 - 5 % of at least one gelling agent,
- 0 - 10 % of a surfactant,
- 0-2.5 % buffer, and
- q.s. water.

The compositions of the invention may be formulated into any form suitable for topical administration without a membrane, such as a gel, a solution (such as an aqueous solution), an ointment, a cream, an emulsion, a foam, or the like.

**Exemplary Modes of Administration**

The compositions may be administered by any means effective to apply the composition to a surface of the skin. For example, the compositions may be applied manually, with an applicator such as a dropper or pipette, an applicator such as a swab, brush, cloth, pad, or sponge, or with any other applicator, such as a solid support comprising paper, cardboard or a laminate material, including material comprising flocked, glued or otherwise fixed fibers. Alternatively, the compositions may be applied as an aerosol or non-aerosol spray, from a pressurized or non-pressurized container. In some embodiments, the compositions are administered in metered doses, such as from a metered dose applicator or from an applicator comprising a single dose of the composition.

**Devices**

One aspect of the invention provides a device for administering the compositions. In one embodiment, the device comprises a reservoir containing the composition and a topical applicator for applying the composition to a surface of the skin.

The reservoir may be of any configuration and any material suitable for containing the composition. For example, the reservoir may be rigid or flexible, may be of a unitary construction (such as a molded material) or may be formed from different pieces secured together, such as by laminating, heat-sealing, gluing, welding, riveting, etc. For example, the reservoir may comprise a rolled wall, two walls substantially parallel joined at the vicinity of their periphery (where the walls may be, for example, flexible/deformable, formed by a thermoformed blister, or rigid), or a bottom wall and a cylindrical wall, or any other configuration suitable for containing the composition. In some embodiments, the reservoir comprises a bag, a pouch, a sachet, a blister, an ampoule, a pipette, a vial, a canister, or a bottle. In some embodiments, the reservoir comprises a deformable wall that is adapted to actuate flow of the composition when deformed. In some embodiments, the reservoir is adapted to contain a single dose of the composition.

As used herein "topical applicator" specifies an applicator of any configuration and any material suitable for applying the composition to a surface of the skin. The topical applicator may be integrally formed with the reservoir, such that the reservoir and topical applicator comprise a unitary construction, or the topical applicator may be detachable

from, or provided separately from, the reservoir.

For example, the topical applicator may comprise a dropper, pipette, swab, brush, cloth, pad, sponge, or any solid support, such as a support comprising paper, cardboard or a laminate material, including material comprising flocked, glued or otherwise fixed fibers. In some embodiments, the applicator is pre-loaded with composition, for example, the applicator may be impregnated with composition, such as with a unit dose of the composition. In other embodiments, the applicator is loaded with composition during use.

Alternatively, the topical applicator may comprise an aerosol or non-aerosol spray device, such as a hand pump. In other embodiments, the topical applicator is an opening that permits the product to be dispensed therethrough. In some embodiments, the opening is provided with a removable and replacable device for closing and opening the opening, such as a cap, stopper or plug, which can be placed within or over the opening such as by insertion, screwing, snapping, fitting, or otherwise. In another embodiment, the opening is provided with a removable and disposable device for opening the opening, such as any removable or secable, frangible, peelable or tearable covering over the opening. In other embodiments, the opening is provided with a nozzle or valve, such as a metered dose valve.

In some embodiments, the topical applicator is adapted to dispense a metered dose of the composition, such as a unit dose of a therapeutically effective amount of the composition. In some embodiments, the topical applicator is not a syringe, and the device does not comprise a syringe for intravenous administration.

In some embodiments, the device comprises a single reservoir. In other embodiments, the device contains two or more reservoirs, where each reservoir may contain a single dose of the composition, or may contain any amount of the composition. In some embodiments, the device comprises a single applicator for applying composition from two or more reservoirs. In other embodiments, the device comprises one applicator for applying composition from each reservoir.

In some embodiments, the invention provides a dose, unit dose, or multiple dose of the pharmaceutical composition, such as in a dose package, unit dose package or multiple dose package. In some embodiments, the packaging reflects a dosing regimen or schedule of application, e.g. daily, weekly or twice weekly administration. Advantageously, such packaging of the pharmaceutical composition facilitates accurate application of an amount of the composition, such as a therapeutically effective amount.

According to one embodiment, the composition, device or packet is provided together with instructions for the use thereof in accordance with the methods described herein.

## Methods of Making the Compositions

The invention also relates to a method for making the pharmaceutical composition of the invention. Those skilled in the art can prepare the pharmaceutical compositions of the invention, based on common general knowledge. For example, the bisphosphonate compound can be dissolved in an aqueous phase (e.g., water or buffer) and mixed, followed by addition of the moisturizer and further mixing. A gelling agent, if present, is introduced under stirring. A neutralizer, if present, is added at or near the end of the method, such as to the otherwise final composition. Other optional components can be added at other stages of the method, in accordance with known procedures. For example, a preservative, if present, is added in an appropriate solvent.

## Therapeutic Methods

The present invention also relates to a method for treating a bone-related disorder in a subject in need thereof, comprising administering an effective amount of a pharmaceutical composition according the invention. In one embodiment, the administration is performed by applying an effective amount of the composition of the invention onto a surface of the skin of a patient in need thereof. In some embodiments, the patient to be treated is a mammal, such as a human. The patient may be a male or a female.

In some embodiments, the administration further comprises rubbing the composition into the patient's skin. This rubbing may comprise, for example, gentle rubbing of the composition onto the selected surface area, so that the composition substantially completely penetrates into the patient's skin. In accordance with non film-forming embodiments, the rubbing does not result in the formation of a film on the skin surface.

The administration may follow any suitable administration regimen, as can be determined by those skilled in the art. For example, in one aspect, the method of the invention comprises once daily administration. In another aspect, the method comprises bi-weekly or once-weekly administration. Other suitable regimens are included within the scope of the invention. The present invention also relates to the use of one of the above compositions for the manufacture of a medicament for treating a bone-related disorder.

The term 'treat' or 'treatment' as used herein refers to any treatment of a mammalian condition, disorder, or disease associated with a depressive disorder, and includes, but is not limited to, preventing the condition, disorder, or disease from occurring in a subject which may be predisposed to the condition, disorder, or disease, but has not yet

been diagnosed as having the condition, disorder, or disease; inhibiting the condition, disorder, or disease, for example, arresting the development of the condition, disorder, or disease; relieving the condition, disorder, or disease, for example, causing regression of the condition, disorder, or disease; or relieving the condition caused by the disease or disorder, for example, stopping the symptoms of the disease or disorder. Any such treatment may constitute the achievement of an intended therapeutic effect in a patient.

In some embodiments, the methods and compositions of the invention advantageously result in at least one therapeutic effect selected from the group consisting of reduced fracture frequency, increased bone density, decreased alkaline phosphatase, decreased osteocalcin, decreased N telopeptide collagen I, improved bone architecture, improved bone biomechanical properties (bone strength), for example as can be seen with bending, torsion and/or compression tests, and combinations of one or more of the foregoing therapeutic effects.

The composition and method according to the invention are suitable for treating a bone-related disorder selected from the group consisting of osteoporosis, menopause-associated osteoporosis, glucocorticoid-induced osteoporosis, Paget's disease, abnormal bone resorption, bone cancer, bone loss (generalized bone loss and/or localized bone loss), bone metastasis (with or without hypercalcemia), multiple myeloma and other conditions that feature bone fragility.

**Bioavailability**

The compositions and the methods of the invention can achieve a relative bioavailability of bisphosphonate in the range of 0.1-5%; i.e., can achieve ratios of urinary recovery after dermal administration versus after intravenous (IV) administration in the range of 0.1-5%.

The relative bioavailability of dermally administered bisphosphonate is determined as the ratio of urinary recovery after dermal administration versus urinary recovery after IV administration, as follows:

Relative bioavailability of bisphosphonate (dermal)

= ratio of urinary recovery after dermal administration versus urinary recovery after IV administration

= urinary recovery (dermal) / urinary recovery (IV)

= [Relative amount (%) of administered bisphosphonate recovered in the urine after dermal administration vs. dose administered] / [Relative amount (%) of administered bisphosphonate recovered in the urine after IV administration vs. dose administered]

In one aspect, the compositions and methods of the invention achieve a relative bioavailability of about 0.5%, such as a bioavailability of from 0.1% to 5%. In another aspect, the compositions and methods of the invention achieve a relative bioavailability of 0.1%, 0.25%, 0.5%, 1%, 2%, 3%, 4%, or 5%.

Another measure of bioavailaibilty is urinary excretion. In one embodiment, the compositions and methods of the invention achieve a maximum urinary excretion of about 24 μg (such as 24 μg, or 24 μg +/- 2 μg) of alendronate after a daily therapeutic dermal dose. In another embodiment, the compositions and methods of the invention achieve a maximum urinary excretion of about 63 μg (such as 63 μg, or 63 μg +/- 6 μg) of risedronate after a daily therapeutic dermal dose.

Further advantages of the invention will become apparent from the following examples, which are given below as mere illustrations, and are non limitative.

The skilled person will appreciate that the present invention can incorporate any number of the features described above.

## Examples

### Example 1: Comparative Studies

The solubility of menthol in presence of sodium alendronate or sodium risedronate at 90 % of their solubility in hydroalcoholic mixtures was studied to confirm that compositions such as that described in EP 1 475 095 comprise non-solubilized menthol. The tested compositions contained:

- 10 % w/w absolute ethanol in phosphate buffer at pH 6.0, or
- 20 % w/w absolute ethanol in phosphate buffer at pH 6.0.

### Material and methods

Phosphate buffer at pH 6.0 is prepared as follows. To 250 ml of potassium dihydrogen orthophosphate solution 0.2 M, add 28.5 ml of sodium hydroxide 0.2 M and dilute to 1000.0 ml with water.
The assay is performed by gas chromatography coupled with a FID.
The results are as follows:

| | Mixture containing 10 g ethanol absolute, bisphosphonate at 90 % saturation, QS 100 g phosphate buffer pH 6 | Mixture containing 20 g ethanol absolute, bisphosphonate at 90 % saturation, QS 100 g phosphate buffer pH 6 |
|---|---|---|
| Solubility of menthol in the presence of sodium alendronate | 70 mg / 100 g | 120 mg / 100 g |
| Solubility of menthol in the presence of sodium risedronate | 80 mg/ 100 g | 140 mg / 100 g |

These examples show that menthol has a low solubility in the studied mixtures:

- 10 % w/w absolute ethanol in phosphate buffer at pH 6.0, in the presence of a bisphosphonate at 90% saturation, or
- 20 % w/w absolute ethanol in phosphate buffer at pH 6.0, in the presence of a bisphosphonate at 90% saturation.

Thus, compositions such as the one disclosed in EP 1 475 095 include non-solubilized menthol that would result in crystal formation and/or phase separation upon stopping stirring, and thus are not macroscopically homogenous or stable compositions.

### Example 2: In vitro absorption studies

### Material and methods

**Bisphosphonate compositions**

Radio-labelled ($^{14}C$) alendronic acid (MW 250, anhydrous) or radio-labelled ($^{14}C$) risedronic acid (MW 282, anhydrous) was used in the preparation of pharmaceutical compositions.
Compositions in various vehicles (water/ethanol or buffer/ethanol, with or without additional ingredients, water (pure aqueous)) were prepared, using each bisphosphonate at a concentration of about 90% the saturation value. For example, "90/10" denotes a 90/10 (v/v) mixture of water/ethanol ; "90/10 pH6" denotes a 90/10 (v/v) mixture of phosphate buffer pH6/ethanol.
The additional ingredients tested include: Tween® 80 (T80), oleic acid (OA), menthol, urea, and propylene glycol (PG).
Phosphate buffer at pH 6.0 is prepared as follows. To 250 ml of potassium dihydrogen orthophosphate solution 0.2 M, add 28.5 ml of sodium hydroxide 0.2 M and dilute to 1000.0 ml with water.
Bisphosphonate concentrations are at about 90% saturation.

| Water/ethanol ratio (v/v) | Sodium Alendronate (A), anhydrous concentration (w/w) | Sodium Risedronate (R), anhydrous concentration (w/w) |
|---|---|---|
| 90/10 | 0.88% | 1.66% |
| 80/20 | 0.38% | 0.77% |
| 70/30 | 0.15% | 0.22% |
| 60/40 | 0.06% | 0.17% |
| 50/50 | 0.02% | 0.02% |

**In vitro dermal absorption:**

Principle

In vitro transdermal absorption is quantitatively studied on human ventral dermatomed biopsies placed in a static diffusion cell (Franz cell), according to standard methods. In general terms, dermis is positioned in a Franz cell such that one side of the dermis is in contact with a survival liquid (receptor fluid). The test preparation is applied to the other side of the dermis, and transdermal absorptioned is assessed by measuring the amount of active agent from the test preparation that is detected in the receptor fluid.

Franz Cell Assay

A dermal biopsy is maintained horizontally between two parts of the Franz cell, thus delimiting two compartments:

- one epidermal compartment is comprised of a glass cylinder, having a precisely defined area of 1.77$cm^2$, placed on the upper side of the skin;
- the other dermal compartment is applied to the lower face of the tegument, and comprises a reservoir of fixed volume carrying a lateral collection port.

The two elements are assembled via a clamp.

The lower compartment (dermal) is filled with a receptor liquid constituted of a sodium chloride solution at 9g/L supplemented with bovine serum albumin at 15g/L. At each time point, the survival liquid is entirely sampled out by the lateral collection port and is replaced by fresh liquid.

The lower part of the Franz cell is thermostated at 37˚C. Homogeneity of the temperature and the content in the receptor fluid is maintained by stirring using a magnetic stirrer.

The upper part (epidermal compartment) is open towards the exterior, thus exposing the epidermal surface to the air in the laboratory.

*Preparation of human abdominal dermatomed skin dermal biopsies:*

Skin dermal biopsies are samples from human abdominal skin from plastic surgery. Skin is kept at -20˚C before use. Adherent sub-dermal fat is removed with a scalpel, and skin is brought to a thickness of about 0.5mm with a dermatome.

Franz cells are usually installed the day before application of the test preparation. The epidermal compartment is contacted with the atmosphere in the laboratory, the dermal compartment is thermostated to 37˚C and the skin is contacted with albuminated physiological serum (as described above) for about 17 hours.

The desired amount of test composition is applied with a micropipette onto the whole of the surface of the epidermis delimited by the glass cylinder. To mimic the application of a thin layer of the composition in the *in vivo* setting, a finite dose of 10μL was chosen and applied over 1.77 $cm^2$. Sampling from the liquid contained in the dermal compartment is carried out via the lateral collection port at the desired time point. After 24hrs, following a 5-step washing procedure, epidermis/dermis separation is performed, and the mass balance is calculated.

*Radioactivity measurements*

Detection of radiolabeled bisphophsonate is carried out by liquid scintillation using a particle counter Packard-tricarb 2900 TR.

*Preparation of radioactive samples:*

The receptor liquid sampled from the lower compartment of the Franz cells is directly incorporated in 15mL of liquid scintillation cocktail (Picofluor 40R, Packard) and metered for radioactivity measurement. The epidermis and dermis are digested at 60˚C for a few hours with 1 and 3 ml, respectively, of Soluene 350, Packard. Following digestion, 15 ml of liquid scintillation cocktail (Hionic Fluor, Packard) are added.

*Radioactivity measurements:*

The metering rate is corrected, as far as quenching is concerned, by the method of the external calibration, in order to obtain disintegrations per minute (dpm) accounting for the real activity of each sample. The background is deducted for each sample in cpm. For each scintillation liquid, a specific quenching curve is established.

Results are expressed in weight or percentage of radiolabeled bisphophsonate found in the samples as compared to the administered amount, determined from the metering rates of suitably diluted calibrations.

**Results**

The results of the in vitro dermal absorption assay are presented in Figures 1-13, which are described in more detail below.

Overall, the results demonstrate that the compositions of the invention achieve effective transdermal delivery of bisphosphonates when applied directly to a surface of the skin. Thus, the results support the feasibility of the invention, and demonstrate the performance of the compositions according to the invention, e.g., the ability to administer an effective amount of a bisphosphonate using a topical (dermal) route.

Alendronate may typically be administered orally using a dose of 70 mg (alendronic acid, anhydrous) once a week. Advantageously, for a topical dose of 70 mg anhydrous alendronic acid (equivalent to 76.5 mg of anhydrous sodium alendronate), this corresponds to 7.35 g of a solution according to one embodiment of the invention (alendronate at 90% saturation in buffered hydroalcoholic solution 90/10 buffer/ethanol, i.e. anhydrous monosodium alendronate at 10.4 mg/g). Also according to the invention, the same topical dose of 76.5 mg of anhydrous sodium alendronate corresponds to 2.7 g of another embodiment of the invention (alendronate at 90% saturation in pure water, i.e. anhydrous monosodium alendronate at 28.09 mg/g).

Risedronate sodium is generally administered using a dose of 35 mg of anhydrous monosodium risedronate, once a week. Advantageously, for a topical dose of 35 mg, this corresponds to 1.5 g of a solution according to one embodiment of the invention (risedronate at 90% saturation in phosphate-buffered hydroalcoholic solution 90/10 water/ buffer, i.e. anhydrous monosodium risedronate at 22.4 mg/g). Also according to the invention, the same topical dose of 35 mg, i.e. 0.8 g of another embodiment of the invention (risedronate at 90% saturation in pure water, i.e. anhydrous monosodium risedronate at 45.3 mg/g).

These amounts/volumes of composition are indeed acceptable in the clinical setting. The *in vitro* condition of application - i.e. 10 $\mu$l/1.77 $cm^2$ - mimics an *in vivo* situation wherein the formulation is applied as a thin layer of 1-2 mg of formulation/$cm^2$. Thus, advantageously, the active agent (e.g., the biphosphonate compound) will not be concentrated on a small surface area such as may cause irritation, thus potentially decreasing any local tolerance issue.

Fig. 1 demonstrates that alendronate does cross the skin and is also recovered in the deepest layer of the skin, the dermis. This absorption, expressed as percentage of the dose applied, is not significantly modified by the increase in alcohol content in the solution.

Fig. 2 demonstrates that risedronate does cross the skin and is also recovered in the deepest layer of the skin, the dermis. This absorption, expressed as percentage of the dose applied, is slightly increased by the increase in alcohol content in the solution.

Fig 3. demonstrates that the pH value can be slightly increased by replacement of water with phosphate buffer, in order to reach pH values in the formulation close to skin pH (5.5), without detrimentally affecting absorption.

As seen with Fig. 4, the alcohol content in the formulation does not detrimentally affect the percentage of absorption for alendronate.

Fig. 5 represents the same experiment as in Fig. 4, but the results are expressed in a different way, i.e. as % of the dose in Fig. 4 and as amount in Fig. 5. These figures reveal that the greatest amounts of delivery are obtained with the pure aqueous solution.

Fig. 6 demonstrates that menthol at 90% of its saturating concentration in the 90/10 phosphate buffer/ethanol formulation does not increase the amount of alendronate recovered in the receptor fluid and dermis as compared to pure phosphate buffer solution

Similarly to Fig.6, Fig. 7 demonstrates that menthol does not increase the amount of risedronate recovered in the receptor fluid and dermis as compared to the 100% phosphate buffer solution.

Fig. 8 demonstrates that urea has a neutral effect on the amount recovered in the receptor fluid or the dermis, when incorporated in phosphate buffer solution.

Similarly to Fig. 8, Fig. 9 shows that urea has a neutral effect on the amount of risedronate recovered in the receptor fluid and the skin, when incorporated in the phosphate buffer solution.

Fig. 10 confirms that urea has a neutral effect on the amount of alendronate recovered in the receptor fluid and the skin, and shows that propylene glycol tends to reduce the amount of alendronate recovered as compared to the 100% phosphate buffer solution.

Fig. 11 indicates that urea increases the amount of risedronate in the receptor fluid and the dermis, and that propylene glycol (PG) does not significantly affect the amount of risedronate in the receptor fluid as compared to the 100% phosphate buffer solution.

Fig. 12 shows that glycerine has a neutral effect on the amount of alendronate recovered in the receptor fluid and the dermis. Other data in Fig. 12 reflect results obtained when oleic acid, a known enhancer, was incorporated at 90% of its maximum solubility in a 90/10 phosphate buffer/ethanol solution containing Tween® 80 (T80) at 4.5%. Under those conditions (e.g., with oleic acid), the amount of risedronate recovered in the receptor fluid and the dermis are lower when compared to the 100% phosphate buffer solution.

Fig. 13 shows that glycerine does not significantly increase the amount of risedronate recovered in the receptor fluid and the dermis. Other data in Fig. 13 reflect results obtained when oleic acid, a known enhancer, was incorporated at 90% of its maximum solubility in a 90/10 phosphate buffer/ethanol solution containing Tween® 80 at 4.5%. Under those conditions (e.g., with oleic acid), the amount of risedronate recovered in the receptor fluid and the dermis are lower when compared to the 100% phosphate buffer solution.

## Example 3: Exemplary Compositions

The following compositions can be prepared for use in accordance with the invention:

| Formulation (g per 100g total) | **#1** | **#2** | **#3** | **#4** | **#5** | **#6** |
|---|---|---|---|---|---|---|
| **Alendronate monosodium tryhydrate** | 3.375 | 3.375 | 3.375 | 3.375 | 3.375 | 3.375 |
| **Carbopol® 980 NF** | 0.8 | 1 | - | - | - | - |
| **Carbopol Ultrez® 10** | - | - | 1 | - | - | - |
| **Natrosol© (hydroxyethylcellulose)** | - | - | - | 1.2 | 1.7 | - |
| **Phosphate Buffer pH6** | 93.425 | 92.625 | 92.625 | 95.425 | 94.925 | 96.625 |
| **Trolamine 1/2** | 2.4 | 3 | 3 | - | - | - |
| **Glycerine** | 0-10 | 0-10 | 0-10 | 0-10 | 0-10 | 0-10 |

## Example 4: In vivo absorption studies

### Summary

The purpose of this experiment was to determine the relative bioavailability of $^{14}C$-alendronate in bone after a single dose of $^{14}C$-alendronate administered as either an intravenous bolus of 48 μg (3 rats) or a dermal application of 1.9 mg (5 rats) after a period of 24 hours. The treated skin area was protected with non occlusive gauze that did not affect delivery. After 24 hours, gauze was removed and the skin was washed. Concentration of $^{14}C$-alendronate in bone, treated skin area, plasma, red blood cells and liver was determined. Recovery of excreted $^{14}C$-alendronate was determined from urine and faeces, and the total amount of $^{14}C$-alendronate remaining in the carcass at the termination of the study - Day 4 or Day 8- was determined.

Formulations: Dosing formulations for intravenous administration were prepared by diluting $^{14}C$-alendronate with unlabelled alendronate and dissolving and diluting in normal saline to a concentration of 0.2 mg/ml (specific activity 0.261 μCi/μg). Dosing formulations for dermal administration were prepared by diluting $^{14}C$-alendronate with unlabelled alendronate and dissolving and diluting in phosphate buffer to a concentration of 33.8 mg/ml (specific activity 0.015 μCi/μg).

Animals: Twenty female CD [CRL:CD (SD)] rats were used in this study. At the time of dosing, the rats were 9-11 weeks old and weighed 229-265 g.

Experimental design:

Group 1 animals received intravenous doses of 0.2 mg/kg $^{14}$C-alendronate at a dose volume of 1 ml/kg body weight.

Group 2 animals received dermal administration of 1.9 mg $^{14}$C-alendronate at a dose volume of 0.056 ml. Before dosing, an Elizabethan collar was attached to the Group 2 animals. After drying at ambient temperature, dermally administered $^{14}$C-alendronate was protected for 24 hours with a non occlusive gauze dressing. After 24 hours, the Elizabethan collar and the gauze dressing were removed, the application site was rinsed several times with water and dried with cotton swabs, and the amount of radioactivity in the gauze dressing and cotton swabs was determined.

Blood samples were collected from the intravenously dosed animals at 30 minutes, 1 hour, and 2 hours after dosing. Blood samples from dermally dosed animals were collected at 6, 12, 24, 72, 120 and 168 hours after dosing. Plasma samples were collected separately for determination of radioactivity. Urine and faeces were collected at 0-8 hours, 8-24 hours, and every 24 hours thereafter. At 72 hours (Study Day 4) or 168 hours (Study Day 8) after administration of alendronate, animals were euthanized.

The experimental design is illustrated in the following table:

| Group | Route of Administration | Test Article Dose | Dose Volume | Number of Animals | Number of Animals Sacrificed | |
|---|---|---|---|---|---|---|
| | | | | | Day 3 | Day 7 |
| 1 | Intravenous | 0.2 mg/kg | 1 ml/kg | 6 | 3 | 3 |
| 2 | Dermal | 1.9 mg | 0.056 ml | 10 | 5 | 5 |

Skin Preparation: Approximately 24 hours prior to dermal administration, fur from the trunks of the animals in Group 2 was clipped with a veterinary clipper so that no less than 10% (approximately 24 $cm^2$) of dorsal body surface area was available for application of the test material. Care was taken to avoid abrading the skin. The size of the shaved area encompassed the majority of the dorsal surface area from the scapular (shoulder) region to just above the rump.

Morbidity/Mortality Observations: Animals were observed twice daily during the treatment period for mortality or evidence of morbidity. Mortality/morbidity checks were separated by a minimum of four hours.

Clinical Observations: Detailed clinical observations were performed daily during the study period. The initial clinical observation was done within 30 minutes following the intravenous administration and approximately two hours following dermal administration.

Body Weights: Animals were weighed at receipt (random sample), once during quarantine (randomization), immediately prior to dosing and prior to the scheduled terminal necropsy (fasted weight).

Post-mortem Examination Procedures: All test animals received an abbreviated necropsy. Half the animals in each group (three from Group 1 and five from Group 2) were euthanized on Day 4. The remaining animals were euthanized on Day 8. Rats scheduled for euthanasia were fasted overnight and euthanized by the induction of sodium pentobarbital anaesthesia followed by exsanguination. At necropsy, the liver, femur, tibia and skin (application area for dermally dosed animals) were

collected, weighed and stored at -70 ˚C until determination of radioactivity. The remainder of the carcass and all fluids and excreta also were stored at -70 ˚C until determination of radioactivity.

Determination of Radioactivity: A portion of each tissue or the entire tissue was weighed or measured, and the radioactivity present was determined. Radioactivity (DPM) was measured using a Model 2200A Liquid Scintillation Counter (Perkin-Elmer, Boston, MA). Liver, femur, tibia, red blood cells and faeces were homogenized (Tissue Tearor, Biospec Products, Inc., Bartlesville, OK) with water and oxidized in an OX-500 Biological Material Oxidizer (R.J. Harvey Instrument Corporation, Hillsdale, NJ). Marrow was removed from bone samples prior to homogenization. $^{14}CO_2$ from sample combustion was trapped in Carbon 14 Cocktail (R.J. Harvey) scintillation fluid and radioactivity was counted. Skin samples were cut into pieces of approximately 200 mg each and oxidized completely. Plasma, urine and cage washes were added directly to liquid scintillation cocktail (Scintisafe Plus 50%, Fisher Scientific, Fair Lawn NJ or Optiphase Supermix, Perkin-Elmer, Boston, MA) and radioactivity was counted. Carcasses were homogenized by dissolving in 10 M NaOH at approximately 85 ˚C. An aliquot of the homogenate was neutralized by the addition of glacial acetic acid and colour was removed by addition of $H_2O_2$. An aliquot was added to scintillation fluid or oxidised and radioactivity determined

Data Analysis: The actual amount of $^{14}$C-alendronate administered intravenously was calculated from the volume administered and multiplied by the concentration. Administration of 100% of the intravenous dose was assumed.

For determination of the actual amount of $^{14}$C-alendronate administered dermally, the total amount of the dermal dose (1.9 mg) was adjusted for the amount of radioactivity recovered from the pipettor tip used for dosing. This

value for the dose administered was used for all calculations of recovery.

The $^{14}C$-alendronate in liver, femur, tibia and red blood cells was reported as μg $^{14}C$-alendronate per g tissue. Recovery of $^{14}C$-alendronate from excreta was reported as μg $^{14}C$-alendronate per time period. The amount of $^{14}C$-alendronate recovered in urine and cage washes was determined and reported as a single value. The amount of $^{14}C$-alendronate recovered from the carcass was reported as a total amount of $^{14}C$-alendronate recovered. Background radioactivity was subtracted from all samples using an appropriate blank sample. For oxidized samples, tissues from a control animal were oxidized and the amount of radioactivity was determined.

**Summary of results**

Dose Administration: Gauze wrap removed at 24h contained about 10% of the delivered dose of alendronate while washing medium and swabs contained about 46%. Thus, 56% of the dermally delivered alendronate were not absorbed

Concentrations of $^{14}C$-alendronate in Bone:

After intravenous administration, interindividual variability of bone concentration was minimal, concentration in tibia was similar to concentration in femur, and concentration on Day 8 was slightly higher than on Day 4 After dermal administration, large interindividual variations of bone concentration were observed.

Concentration of $^{14}C$-alendronate in Bone:

Intravenous Administration (Group 1)

| Necropsy Day | Animal Number | μg $^{14}C$-alendronate/g bone | |
|---|---|---|---|
| | | Femur | Tibia |
| Day 4 | 771 | 0.525 | 0.551 |
| | 772 | 0.408 | 0.547 |
| | 773 | 0.568 | 0.379 |
| | Mean | 0.500 | 0.492 |
| | SD | 0.083 | 0.098 |
| | RSD | 17% | 20% |
| Day 8 | 774 | 0.707 | 0.704 |
| | 775 | 0.692 | 0.680 |
| | 776 | 0.639 | 0.374 |
| | Mean | 0.679 | 0.586 |
| | SD | 0.036 | 0.184 |
| | RSD | 5% | 31% |

Dermal Administration (Group 2)

| Necropsy Day | Animal Number | μg $^{14}C$-alendronate/g bone | |
|---|---|---|---|
| | | Femur | Tibia |
| Day 4 | 777 | 0.222 | 0.130 |
| | 778 | 0.018 | 0.039 |
| | 779 | 0.018 | 0.016 |
| | 780 | 0.015 | 0.002 |
| | 781 | 0.026 | 0.011 |

(continued)

Dermal Administration (Group 2)

| Necropsy Day | Animal Number | μg $^{14}C$-alendronate/g bone | |
|---|---|---|---|
| | | Femur | Tibia |
| | Mean | 0.060 | 0.039 |
| | SD | 0.091 | 0.053 |
| | RSD | 152% | 134% |
| Day 8 | 782 | 0.022 | 0.012 |
| | 783 | 0.016 | ND |
| | 784 | 0.011 | 0.007 |
| | 785 | ND | ND |
| | 786 | 0.069 | 0.031 |
| | Mean | 0.029 | 0.017 |
| | SD | 0.027 | 0.012 |
| | RSD | 92% | 74% |

Recovery of $^{14}C$-alendronate from Skin:

The results are presented below and demonstrate that alendronate retention in the skin shows small inter variability, is minimal, thus potentially minimising any local tolerance issue.

Recovery of $^{14}C$-alendronate from Treated Skin Dermal Administration (Group 2) Only

| Necropsy Day | Animal Number | Dose (μg) | Amount in Skin Sample (μg) | % of Dose |
|---|---|---|---|---|
| Day 4 | 777 | 1885 | 6.17 | 0.35% |
| | 778 | 1890 | 5.49 | 0.30% |
| | 779 | 1871 | 6.68 | 0.35% |
| | 780 | 1876 | 8.21 | 0.44% |
| | 781 | 1886 | 6.19 | 0.33% |
| | Mean | | 6.55 | 0.35% |
| | SD | | 1.02 | 0.05% |
| | RSD | | 16% | |
| Day 8 | 782 | 1883 | 4.70 | 0.25% |
| | 783 | 1890 | 4.12 | 0.22% |
| | 784 | 1886 | 6.13 | 0.33% |
| | 785 | 1884 | 7.32 | 0.39% |
| | 786 | 1869 | 4.04 | 0.22% |
| | Mean | | 5.26 | 0.28% |
| | SD | | 1.43 | 0.08% |
| | RSD | | 27% | |

Excretion of $^{14}C$-alendronate: The amount of $^{14}C$-alendronate excreted in urine and faeces samples are presented below. Approximately 7% of the dose was excreted in the urine by 168 hours after intravenous administration. Most of this (approximately 5% of the dose) was excreted in the first eight hours. After dermal administration, approximately 0.4% of the dose was excreted in the urine by 168 hours. After intravenous administration, approximately 6% of the dose was excreted in the faeces by 168 hour. After dermal administration, approximately 2% of the dose was excreted

in the faeces by 168 hours.

Urinary Excretion of $^{14}C$-alendronate - Intravenous Administration (Group 1)

| Animal | Amount Excreted (% of Dose) | | | | | | | | Total Excretion (% of Dose) | |
|---|---|---|---|---|---|---|---|---|---|---|
| Number | 8 hours | 24 hours | 48 hours | 72 hours | 96 hours | 120 hours | 144 hours | 168 hours | 0-72 hours | 0-168 hours |
| 771 | 5.02% | 1.28% | 0.94% | 0.34% | -[a] | - | - | - | 7.6% | - |
| 772 | 9.11% | 0.94% | 0.75% | 0.51% | - | - | - | - | 11.3% | - |
| 773 | 4.88% | 1.37% | 0.95% | 0.61% | - | - | - | - | 7.8% | - |
| 774 | 2.05% | 1.27% | 0.34% | 0.20% | 0.15% | 0.11% | 0.06% | 0.18% | 3.9% | 4.4% |
| 775 | 3.01% | 1.06% | 0.85% | 0.26% | 0.57% | 0.27% | 0.31% | 0.16% | 5.2% | 5.5% |
| 776 | 5.49% | 1.19% | 0.78% | 0.56% | 0.74% | 0.43% | 0.59% | 0.33% | 8.0% | 10.1% |
| Mean | 4.9% | 1.2% | 0.8% | 0.4% | 0.5% | 0.3% | 0.3% | 0.2% | 7.3% | 7.0% |
| SD | 2.4% | 0.2% | 0.2% | 0.2% | 0.3% | 0.2% | 0.3% | 0.1% | 2.6% | 2.9% |
| [a] Animal was necropsied on Day 4. | | | | | | | | | | |

Urinary Excretion of $^{14}C$-alendronate - Dermal Administration (Group 2)

| Animal | Amount Excreted (% of Dose) | | | | | | | | Total Excretion (% of Dose) | |
|---|---|---|---|---|---|---|---|---|---|---|
| Number | 8 hours | 24 hours | 48 hours | 72 hours | 96 hours | 120 hours | 144 hours | 168 hours | 0-72 hours | 0.168 hours |
| 777 | 0.14% | 0.33% | 0.14% | 0.13% | -[a] | - | - | - | 0.74% | - |
| 778 | 0.09% | 0.07% | 0.02% | 0.02% | - | - | - | - | 0.21% | - |
| 779 | 0.08% | 0.06% | 0.06% | 0.03% | - | - | - | - | 0.23% | |
| 780 | 0.83% | 0.16% | 0.12% | 0.06% | - | - | - | - | 1.16% | |
| 781 | 0.07% | 0.11% | 0.03% | 0.01% | - | - | - | - | 0.22% | |
| 782 | 0.15% | 0.07% | 0.06% | 0.06% | 0.05% | 0.06% | 0.02% | 0.01% | 0.34% | 0.48% |
| 783 | 0.02% | 0.06% | 0.06% | 0.02% | 0.03% | 0.02% | 0.01% | 0.00% | 0.16% | 0.22% |
| 784 | 0.05% | 0.07% | 0.06% | 0.03% | 0.02% | 0.01% | 0.01% | 0.00% | 0.20% | 0.24% |
| 785 | 0.08% | 0.05% | 0.02% | 0.01% | 0.01% | 0.01% | 0.01% | 0.00% | 0.17% | 0.19% |
| 786 | 0.22% | 0.27% | 0.06% | 0.02% | 0.03% | 0.02% | 0.01% | 0.00% | 0.57% | 0.62% |
| Mean | 0.17% | 0.12% | 0.06% | 0.04% | 0.03% | 0.02% | 0.01% | 0.00% | 0.40% | 0.35% |
| SD | 0.24% | 0.10% | 0.04% | 0.03% | 0.01% | 0.02% | 0.00% | 0.00% | 0.33% | 0.19% |

Feces Excretion of $^{14}C$-alendronate - Intravenous Administration (Group 1)

| Animal | Amount Excreted (% of Dose) | | | | | | | | Total Excretion (% of Dose) | |
|---|---|---|---|---|---|---|---|---|---|---|
| Number | 8 hours | 24 hours | 48 hours | 72 hours | 96 hours | 120 hours | 144 hours | 168 hours | 0-72 hours | 0-168 hours |
| 771 | 0.02% | 4.14% | 4.44% | NS | - | - | - | - | 8.6% | - |
| 772 | 0.00% | 1.91% | 0.47% | 0.15% | - | - | - | - | 2.4% | - |

(continued)

| Animal | Amount Excreted (% of Dose) | | | | | | | | Total Excretion (% of Dose) | |
|---|---|---|---|---|---|---|---|---|---|---|
| Number | 8 hours | 24 hours | 48 hours | 72 hours | 96 hours | 120 hours | 144 hours | 168 hours | 0-72 hours | 0-168 hours |
| 773 | 0.00% | 1.83% | 1.36% | 0.24% | - | - | - | - | 3.2% | - |
| 774 | 0.00% | 1.38% | 0.92% | 0.44% | 0.14% | 0.12% | 0.08% | 0.02% | 2.3% | 3.1% |
| 775 | 0.67% | 2.19% | 2.10% | 0.87% | 1.17% | 0.54% | 0.59% | 0.05% | 5.0% | 8.2% |
| 776 | 0.01% | 1.03% | 2.09% | 1.17% | 0.32% | 0.56% | 0.36% | 0.06% | 3.1% | 5.6% |
| Mean | 0.12% | 2.08% | 1.89% | 0.57% | 0.55% | 0.41% | 0.34% | 0.04% | 4.1% | 5.6% |
| SD | 0.27% | 1.09% | 1.40% | 0.44% | 0.55% | 0.25% | 0.25% | 0.02% | 2.4% | 2.5% |

Faeces Excretion of $^{14}$C-alendronate - Dermal Administration (Group 2)

| Animal | Amount Excreted (% of Dose) | | | | | | | | Total Excretion (% of Dose) | |
|---|---|---|---|---|---|---|---|---|---|---|
| Number | 8 hours | 24 hours | 48 hours | 72 hours | 96 hours | 120 hours | 144 hours | 168 hours | 0-72 hours | 0-168 hours |
| 777 | 0.007% | | 2.55% | 0.17% | -[a] | - | - | - | 2.73% | - |
| 778 | 0.002% | 0.018% | 0.53% | 0.05% | - | - | - | - | 0.59% | - |
| 779 | 0.001% | 0.001% | 1.40% | | - | - | - | - | 1.40% | |
| 780 | 0.014% | 0.066% | 1.62% | 0.02% | - | - | - | - | 1.71% | |
| 781 | 0.003% | 0.721% | 0.52% | 0.02% | - | - | - | - | 1.27% | |
| 782 | 0.004% | 0.039% | 0.68% | 0.33% | 0.13% | 0.13% | 0.07% | 0.01% | 1.05% | 1.39% |
| 783 | 0.000% | 0.047% | 0.30% | 0.19% | 0.05% | 0.05% | 0.04% | 0.00% | 0.53% | 0.66% |
| 784 | 0.000% | 0.146% | 0.58% | 0.15% | 0.09% | 0.05% | 0.04% | 0.01% | 0.88% | 1.06% |
| 785 | 0.001% | 1.868% | 0.60% | 0.10% | 0.05% | 0.02% | 0.03% | 0.01% | 2.56% | 2.67% |
| 786 | 0.004% | 0.108% | 2.88% | 0.46% | 0.18% | 0.10% | 0.09% | 0.00% | 3.45% | 3.82% |
| Mean | 0.004% | 0.335% | 1.17% | 0.16% | 0.10% | 0.07% | 0.05% | 0.00% | 1.62% | 1.92% |
| SD | 0.004% | 0.617% | 0,92% | 0.15% | 0.05% | 0.05% | 0.03% | 0.00% | 0.99% | 1.30% |

**Example 5: Feasibility Study Based On In Vitro Data (Example 2)**

The in vitro results from the human skin Franz cell experiments described in Example 2 were used to confirm that therapeutically effective amounts of bisphosphonate can be delivered using the compositions and methods of the invention.

As shown in Example 2, alendronate exhibits an absorption rate of 0.6% in vitro. Assuming a 0.7% topical (dermal) absorption through human skin in vivo, and taking into account that approximately 50% of the systemic dose in humans is recovered in the urine, the relative dermal bioavailability of alendronate would be 0.7% X 50% = 0.35%, e.g., about half the relative oral bioavailability of alendronate in human subjects.

Thus, this model indicates that in order to dermally deliver an amount of alendronate equivalent to an oral dose, one would dermally administer twice the oral dosage. Thus, for example, to dermally deliver an amount of alendronate equivalent to an oral weekly dose of 70 mg of alendronate, one would dermally administer once weekly twice that amount, i.e. 2 x 70 mg = 140 mg. For a composition of the invention having an alendronate concentration of 33.3 mg/g, this would correspond to dermally administering once weekly about 4g (such as 4g) of the composition, or twice weekly about 2g (such as 2g) of the composition. Such amounts are easily administered dermally, thus confirming that therapeutically effective amounts of bisphosphonate can be delivered using the compositions and methods of the invention.

**Example 6: Feasibility Study Based On Urinary Recovery (Example 4)**

The in vivo results from the urinary recovery experiments described in Example 4 were used to confirm that therapeutically effective amounts of bisphosphonate can be delivered using the compositions and methods of the invention.

As explained above, relative bioavailability is determined using urinary recovery after IV administration as a reference. Thus, relative bioavailability after oral administration is determined as follows:

Relative bioavailability (oral)

= ratio of urinary recovery after oral administration versus ratio of urinary recovery after IV administration

= urinary recovery (oral) / urinary recovery (IV)

= [Relative amount (%) of administered bisphosphonate recovered in the urine after oral administration vs. oral dose administered] / [Relative amount (%) of administered bisphosphonate recovered in the urine after IV administration vs. IV dose administered]

According to the literature (e.g., J.H. Lin, G. Russel, B.Gertz "Pharmacokinetics of alendronate: an overview" Int J Clin Pract Suppl 1999, 101, p18-26), alendronate shows a relative oral bioavailability of 0.7% in human subjects. Using data from Example 4 showing that urinary recovery after dermal administration is 0.4% in the rat, and published data showing that urinary recovery after IV administration in the rat is 36% (e.g., J.H. Lin, G. Russel, B.Gertz "Pharmacokinetics of alendronate : an overview" Int J Clin Pract Suppl 1999, 101, p18-26) (note that this amount differs from the amount determined in Example 4), the relative dermal bioavailability of alendronate in the rat (dermal vs. IV) would be 0.4% / 36% = 1.1%. Based upon in vitro results showing a 10-fold higher absorption in rats than in humans, one may reasonably assume that the in vivo dermal absorption in the rat is 10-fold higher than in humans. Therefore, the relative dermal bioavailability of alendronate in humans (dermal vs. IV) would be 10-fold lower than that of the rat, i.e. 1.1 % / 10 = 0.1 %.

Comparing the relative dermal bioavailability of alendronate in humans (0.1% dermal vs. IV) to the relative oral bioavailability of alendronate in humans (0.7%, oral vs. IV), suggests that relative dermal bioavailability in humans is 7-fold lower than relative oral bioavailability. Thus, this model indicates that in order to dermally administer an amount of alendronate equivalent to an oral dose, one would dermally administer seven times the oral dosage. Thus, for example, to dermally deliver an amount of alendronate equivalent to an oral weekly dose of 70 mg of alendronate, one would dermally administer once weekly seven times that amount, i.e. 7 x 70 mg = 490 mg. For a composition of the invention having an alendronate concentration of 33.3 mg/g, this would correspond to dermally administering once weekly about 14.5g (such as 14.5g) of the composition, or twice weekly about 7g (such as 7g) of the composition. Such amounts are easily administered dermally, thus confirming that therapeutically effective amounts of bisphosphonate can be delivered using the compositions and methods of the invention.

**Example 7 : Feasibility Study Based Upon Bone Recovery (Example 4)**

The in vivo results from the bone recovery experiments described in Example 4 were used to confirm that therapeutically effective amounts of bisphosphonate can be delivered using the compositions and methods of the invention.

Using data from Example 4 showing that bone recovery after dermal administration is 0.2% in the rat, and published data showing that bone recovery after oral administration in the rat is 0.9% (J.H. Lin et al., "on the absorption

of alendronate in rats," J Pharm Sci 1194 83(12), p1741-46), it appears that dermal administration results in about 5-fold lower bone bioavailability than oral administration, in the rat: bone recovery in rat after oral vs. dermal administration = 0.9% / 0.2% = about 5.

Assuming that the ratio of bone recovery after oral vs. dermal administration is similar in human subjects (e.g., about 5), the relative dermal bone bioavailability of alendronate in humans would be about 5-fold lower than the relative oral bone bioavailability. Thus, this model indicates that in order to dermally administer an amount of alendronate equivalent to an oral dose, one would dermally administer five times the oral dosage. Thus, for example, to dermally deliver an amount of alendronate equivalent to an oral weekly dose of 70 mg of alendronate, one would dermally administer once weekly five times that amount, i.e. 5 x 70 mg = 350 mg. For a composition of the invention having an alendronate concentration of 33.3 mg/g, this would correspond to dermally administering once weekly about 10g (such as 10g) of the composition, or twice weekly about 5g (such as 5g) of the composition. Such amounts are easily administered dermally, thus confirming that therapeutically effective amounts of bisphosphonate can be delivered using the compositions and methods of the invention.

As noted above, those skilled in the art can use any one of the foregoing models, or other means known in the art, to determine an appropriate amount of composition to administer to achieve the intended therapeutic effect.

While the invention has been described and exemplified in sufficient detail for those skilled in this art to make and use it, various alternatives, modifications, and improvements should be apparent without departing from the spirit and scope of the invention. The examples provided herein are representative of specific embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by specific embodiments and optional features, modification and variation of the concepts herein disclosed may be resorted to by those skilled in the art, and that such modifications and variations are considered to be within the scope of this invention as defined by the appended claims.

## Claims

**1.** A liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

**2.** The liquid pharmaceutical composition of claim 1, wherein the composition is a stable, macroscopically homogenous solution.

**3.** The liquid pharmaceutical composition of any one of claims 1 or 2, consisting essentially of a therapeutically effective amount of at least one bisphosphonate and water.

**4.** The liquid pharmaceutical composition of any one of claims 1-3, comprising an amount (w/w) of bisphosphonate in its free acid form selected from 0.05 - 7.5 %, 0.1-6%, 0.2-5%, 0.5-4.5%, 0.75-4%, 1-3%, or 1.5-2.5%, or an equivalent amount of bisphosphonate salt.

**5.** The liquid pharmaceutical composition of any one of claims 1-4, comprising alendronate at 90% saturation in pure water.

**6.** The liquid pharmaceutical composition of any one of claims 1-5, comprising anhydrous monosodium alendronate at 28.09 mg/g composition.

**7.** The liquid pharmaceutical composition of any one of claims 1-4 comprising an amount (w/w) of alendronate as a monosodium salt trihydrate selected from 0.05-3.8%, 0.1-3.75%, 0.5-3.75%, 0.75-3.75%, 1-3.75%, 1.5-3.75%, 2-3.75%, 2.5-3.75%, 3-3.75%, or 3.25-3.75%.

**8.** The liquid pharmaceutical composition of any one of claims 1-4, comprising risedronate at 90% saturation in pure water.

**9.** The liquid pharmaceutical composition of any one of claims 1-4 or 8, comprising anhydrous monosodium risedronate at 45.3 mg/g composition.

**10.** The liquid pharmaceutical composition of any one of claims 1-4 comprising an amount (w/w) of risedronate as a monosodium salt hemipentahydrate selected from 0.05-5.9%, 0.1-5.9%, 0.5-5.9%, 0.75-5.9%, 1-5.9%, 2-5.9%, 3-5.9%, 3.5-5.9%, 4-5.9%, 4.5-5.9%, 4.75-5.9%, 5-5.9%, or 5.5-5.9%.

**11.** A unit dose package comprising a therapeutically effective amount for topical administration of the liquid pharmaceutical composition of any one of claims 1-10.

**12.** A device comprising:

(A) a reservoir containing a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface, and

(B) a topical applicator.

**13.** The device of claim 12, wherein the reservoir contains a unit dose of a therapeutically effective amount of at least one bisphosphonate.

**14.** The device of claim any one of claims 12-13, wherein the applicator is a metered dose applicator.

**15.** The device of claim 14, wherein the metered dose applicator is adapted to dispense, as each metered dose, a unit dose of a therapeutically effective amount of at least one bisphosphonate.

**16.** The device of any one of claims 12-15, wherein the applicator comprises an applicator selected from the group consisting of a dropper, pipette, swab, brush, cloth, pad, sponge, and solid support.

**17.** The device of any one of claims 12-15, wherein the applicator comprises an applicator selected from the group consisting of an aerosol or non-aerosol spray device.

**18.** The device of any one of claims 12-15, wherein the applicator comprises an opening provided with a removable device for opening the opening.

**19.** The device of claim 18, wherein the applicator comprises an opening provided with a nozzle or valve.

**20.** A device according to any one of claims 12-19, wherein the reservoir comprises a liquid pharmaceutical composition according to any one of claims 1-10.

**21.** A method of administering a therapeutically effective amount of at least one bisphosphonate to a patient in need thereof, comprising topically administering to a surface of skin of the patient a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

**22.** A method for treating a bone-related disorder, comprising topically administering to a surface of skin of a patient in need thereof an effective amount of a liquid pharmaceutical composition comprising:

(i) a therapeutically effective amount of at least one bisphosphonate,
(ii) optionally, a non-irritating amount of at least one moisturizer,
(iii) optionally, at least one short-chain aliphatic alcohol,
(iv) optionally, at least one gelling agent,
(v) optionally, at least one surfactant, and
(vi) water,

wherein said composition:

is a stable, macroscopically homogeneous mixture,
has a pH of between 4.0 and 8.5,
is non-occlusive and non film-forming, and
is adapted for topical administration to a skin surface.

**23.** The method according to claim 22, wherein said bone-related disorder is selected from the group consisting of osteoporosis, menopause-associated osteoporosis, glucocorticoid-induced osteoporosis, Paget's disease, abnormal bone resorption, bone cancer, generalized bone loss, localized bone loss, bone metastasis with or without hypercalcemia, multiple myeloma and other conditions of bone fragility.

**24.** Method according to any one of claims 22-23, wherein said method results in at least one therapeutic effect selected from the group consisting of reduced fracture frequency, increased bone (mineral) density, decreased alkaline phosphatase, osteocalcin, decreased N telopeptide collagen I, improved bone architecture, improved bone biomechanical properties (bone strength), for example as can be seen with bending, torsion and/or compression tests,

and combinations thereof.

**25.** The method according to any one of claims 21-24, wherein the administering results in a ratio of urinary recovery after dermal administration versus intravenous administration of from 0.1- 5%.

**26.** The method according to any one of claims 21-25, comprising administering a composition according to any one of claims 1-10.

**27.** Use of a bisphosphonate in the manufacture of a medicament for treating and/or preventing a bone-related disorder, wherein said medicament is a composition according to any one of claims 1-10.

**FIGURE 1**

PERCENTAGE OF ALENDRONATE RECOVERED IN THE RECEPTOR FLUID AND THE DERMIS AT 24HRS (N=6, 2 SKINS)

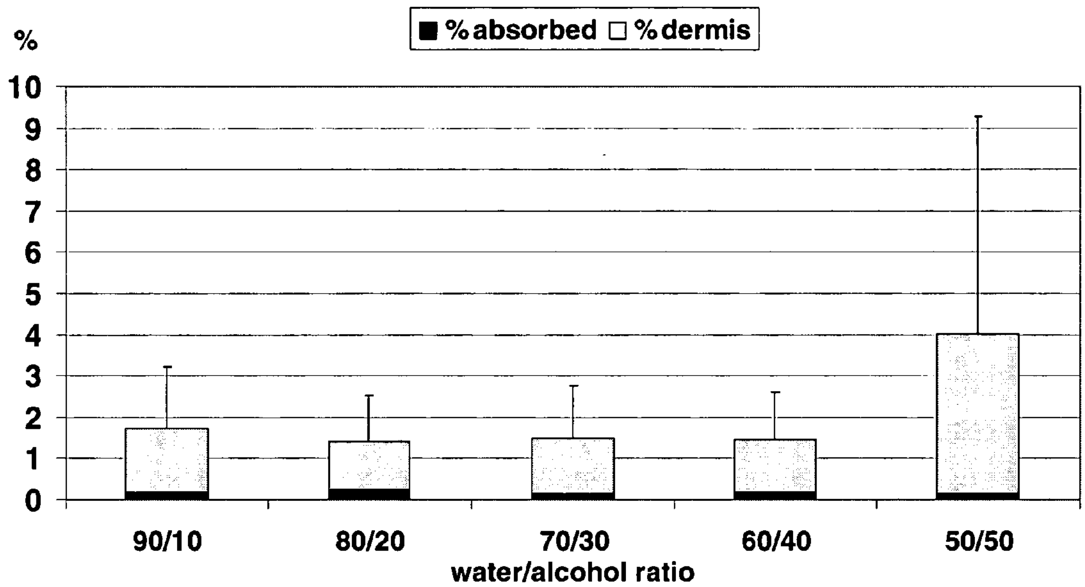


**FIGURE 2**

PERCENTAGE OF RISEDRONATE RECOVERED IN THE RECEPTOR FLUID AND THE DERMIS AT 24HRS (N=6, 2 SKINS)

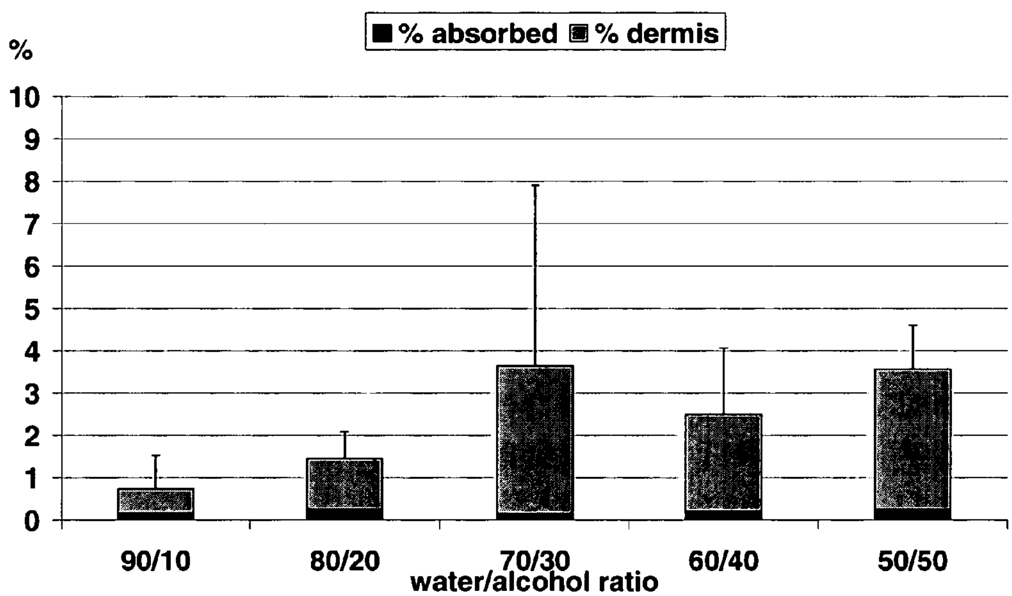

**FIGURE 3**

REPLACEMENT OF WATER BY PHOSPHATE BUFFER PH6 IN SOLUTIONS WITH 10% OF ETHANOL (N=7-8, 3 SKINS)

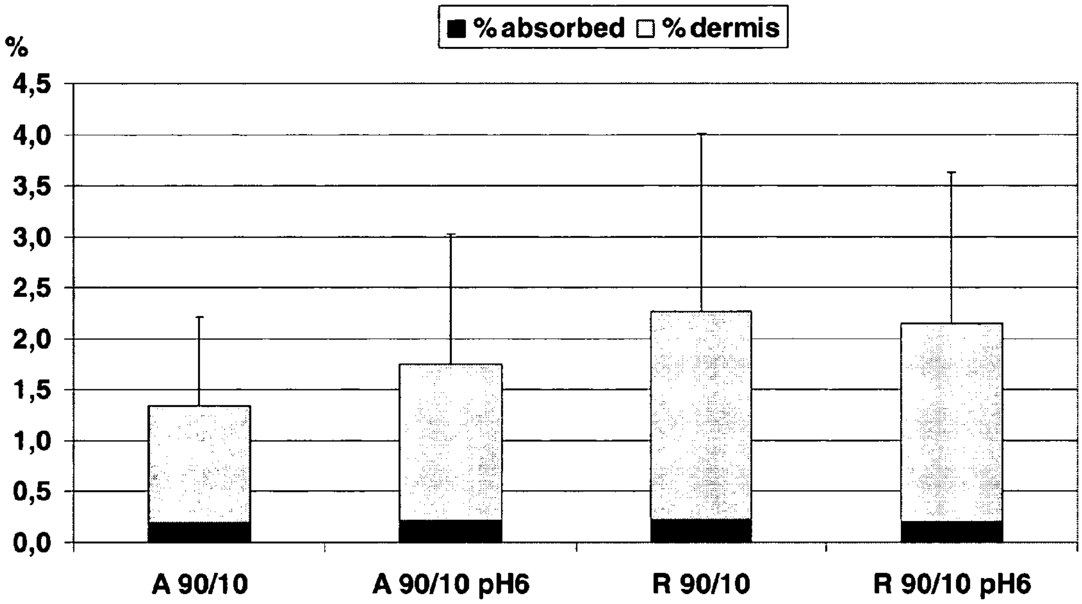


**FIGURE 4**

COMPARISON OF ETHANOL/WATER SOLUTION WITH PURE AQUEOUS SOLUTIONS (N=12; 3 SKINS)

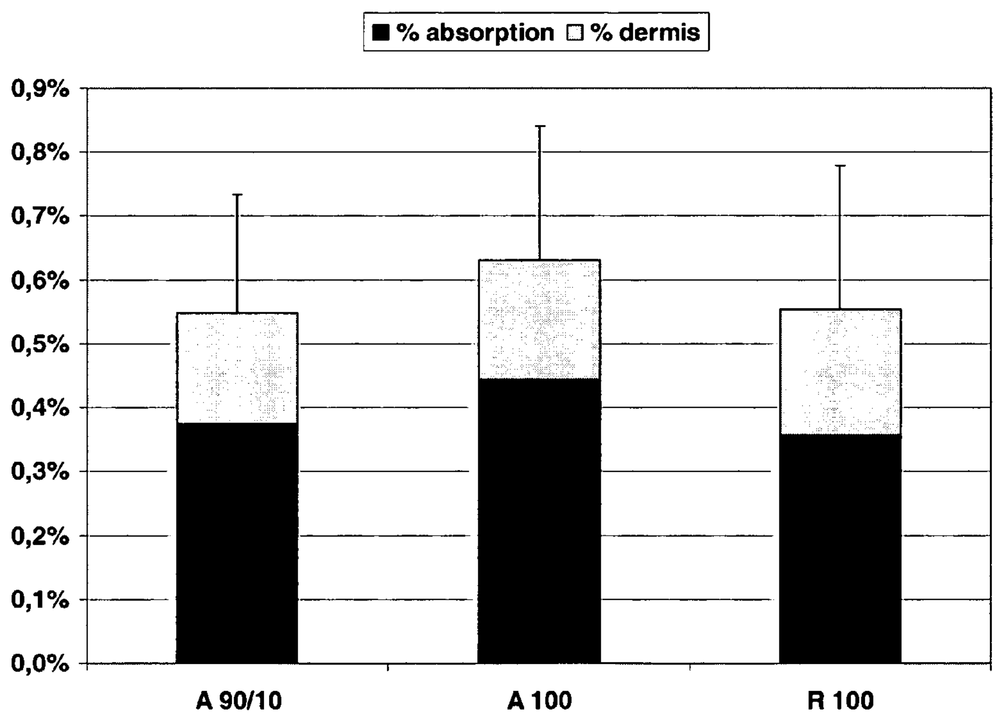

**FIGURE 5**

COMPARISON OF ETHANOL/WATER SOLUTION WITH PURE AQUEOUS SOLUTIONS (N=12; 3 SKINS)

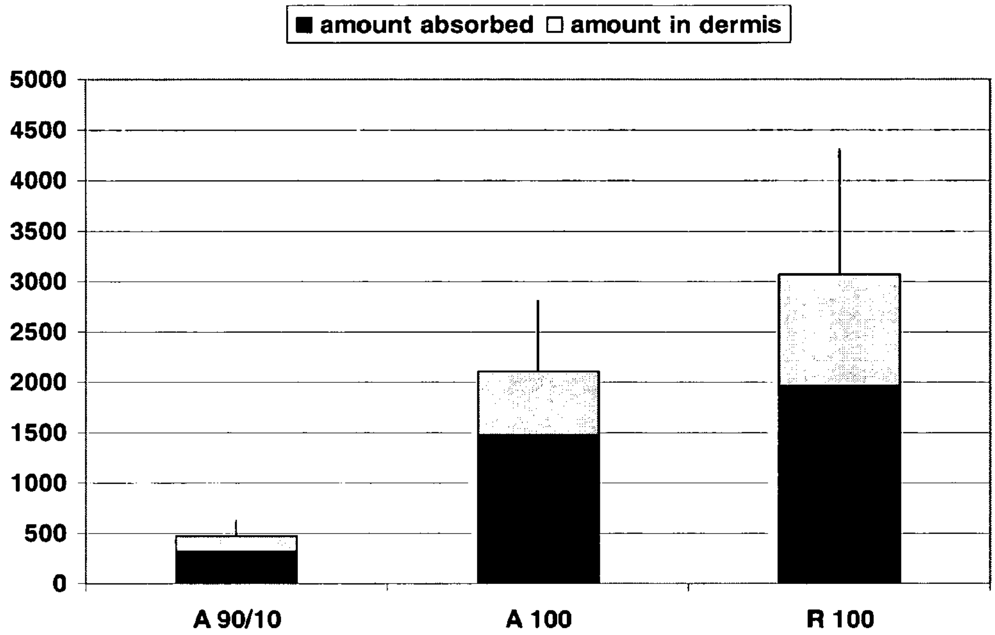


**FIGURE 6**

EFFECT OF MENTHOL ON PERCUTANEOUS ABSORPTION OF ALENDRONATE IN BUFFERED HYDROALCOHOLIC SOLUTION (N=8 OR 6; 3 SKINS)

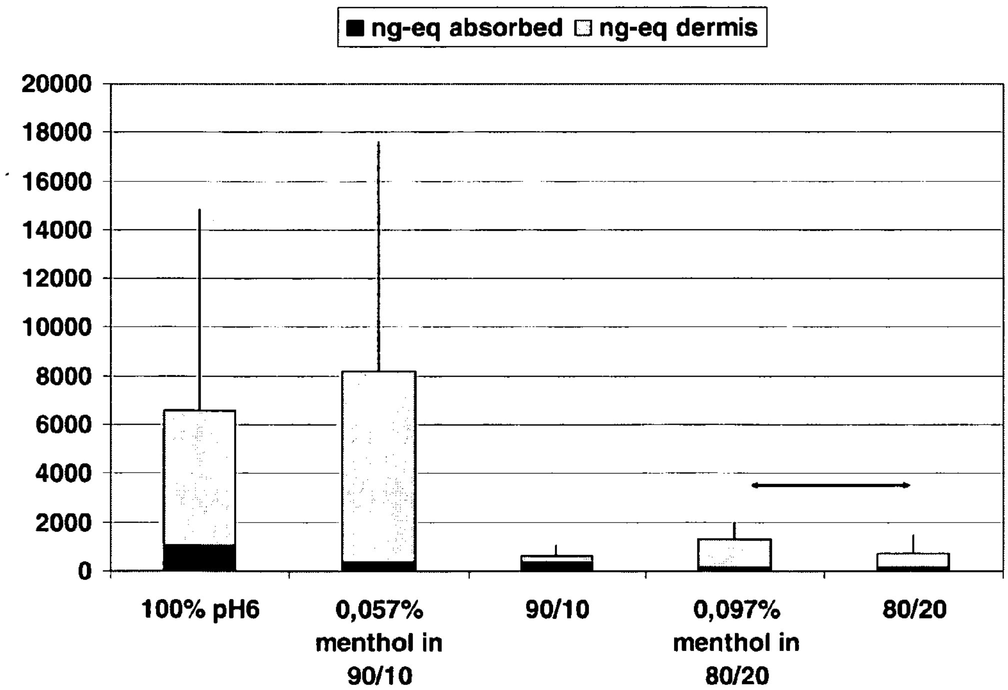

## FIGURE 7

EFFECT OF MENTHOL ON PERCUTANEOUS ABSORPTION OF RISEDRONATE IN BUFFERED HYDROALCOHOLIC SOLUTION (N=6 OR 8; 3 SKINS)

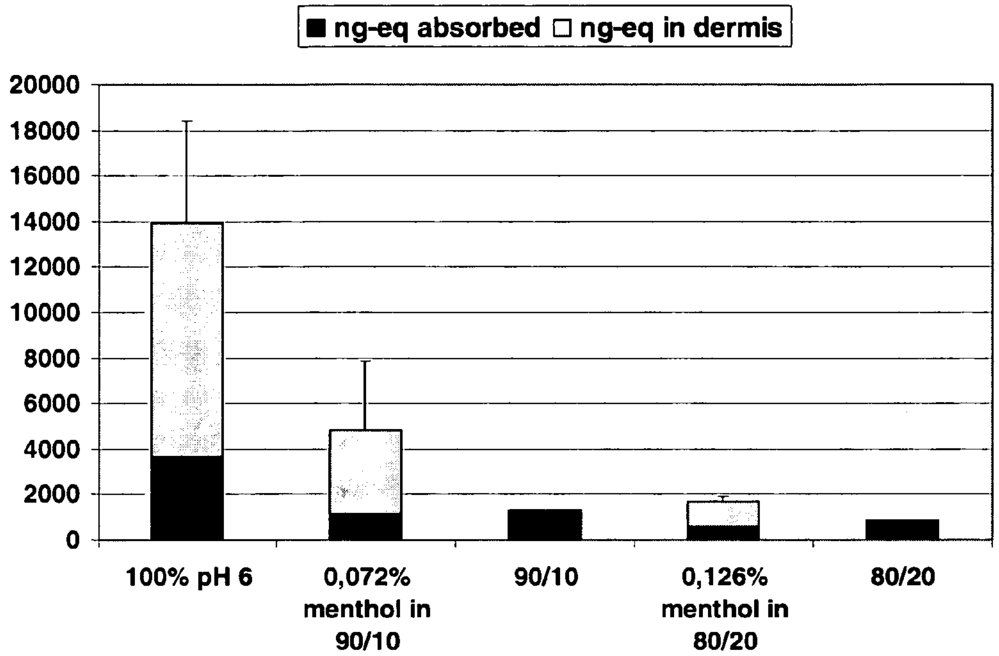


## FIGURE 8

EFFECT OF UREA ON PERCUTANEOUS ABSORPTION OF ALENDRONATE IN BUFFERED HYDROALCOHOLIC SOLUTION (N=8 OR 7; 2 SKINS)

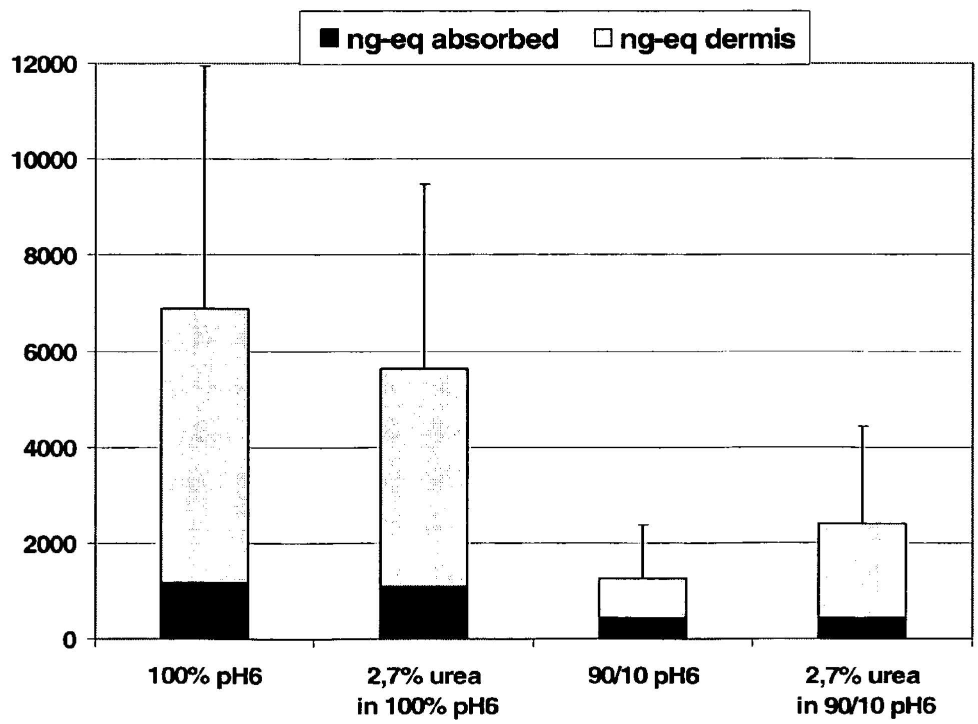

## FIGURE 9

EFFECT OF UREA ON PERCUTANEOUS ABSORPTION OF RISEDRONATE IN BUFFERED HYDROALCOHOLIC SOLUTION (N=8; 2 NON-MOISTURIZED SKINS)

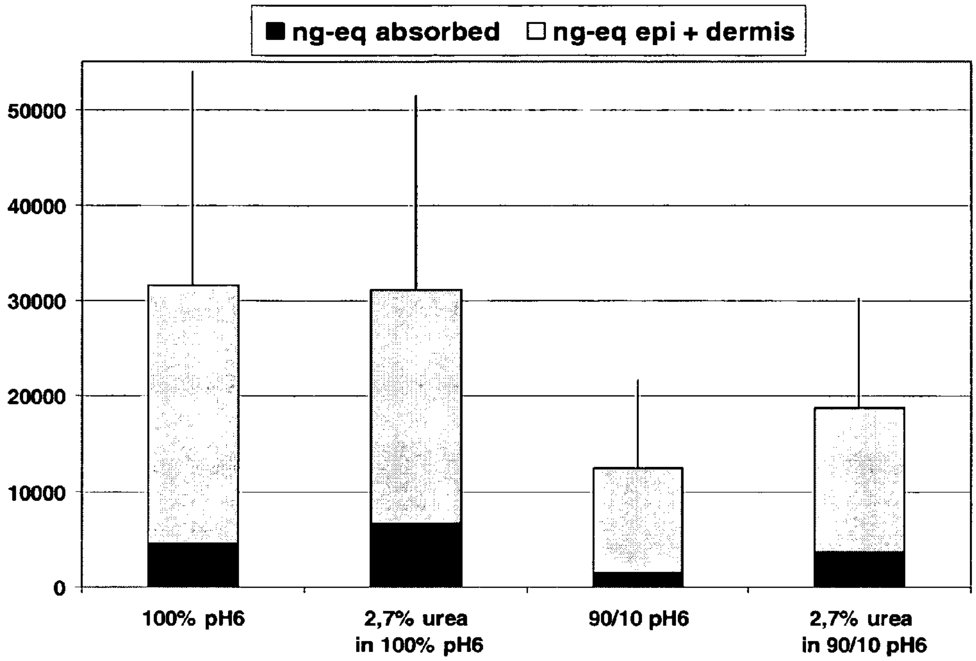


## FIGURE 10

EFFECT OF UREA AND PROPYLENE GLYCOL ON PERCUTANEOUS ABSORPTION OF ALENDRONATE IN BUFFERED SOLUTION (N=8 OR 6; 2 NON-MOISTURIZED SKINS)

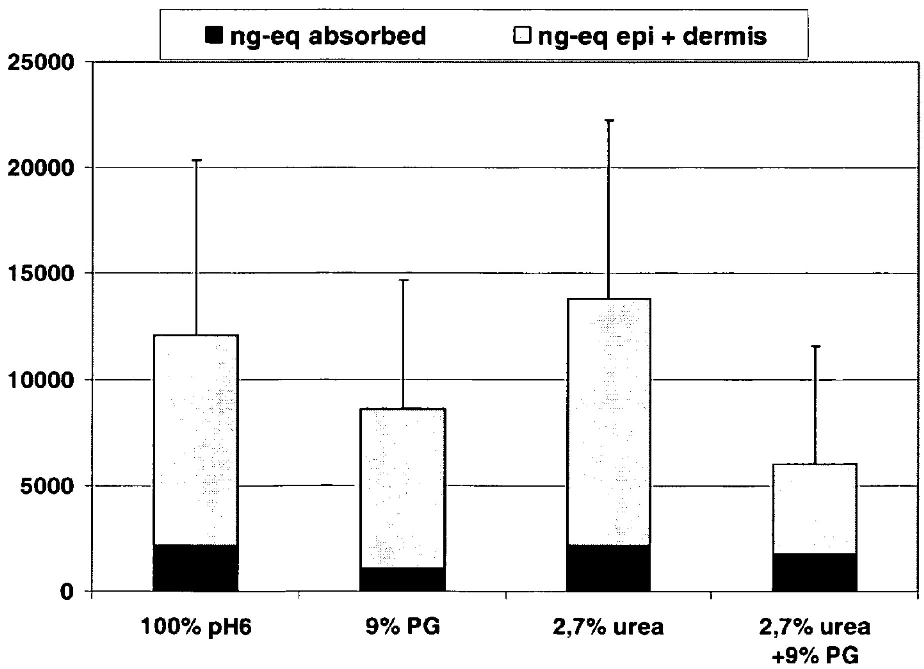

## FIGURE 11

EFFECT OF UREA AND PROPYLENE GLYCOL ON PERCUTANEOUS ABSORPTION OF RISEDRONATE IN BUFFERED SOLUTION (N=8 OR 7; 2 NON-MOISTURIZED SKINS)

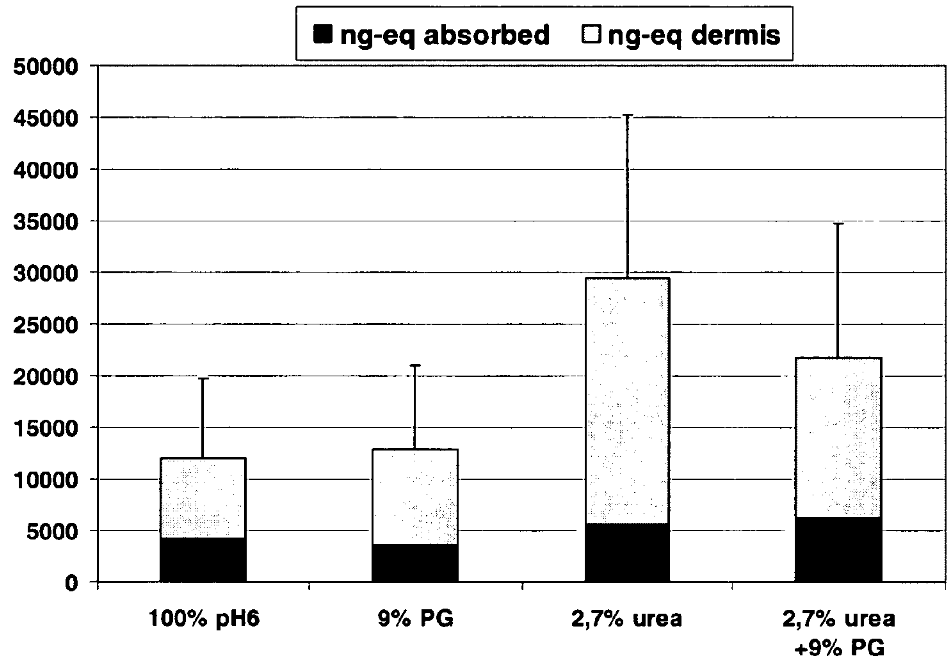


## FIGURE 12

EFFECT OF OLEIC ACID IN THE PRESENCE OF T80 AND OF GLYCERINE ON PERCUTANEOUS ABSORPTION OF ALENDRONATE IN BUFFERED HYDROALCOHOLIC SOLUTION (N=6 - 8; 2 SKINS)

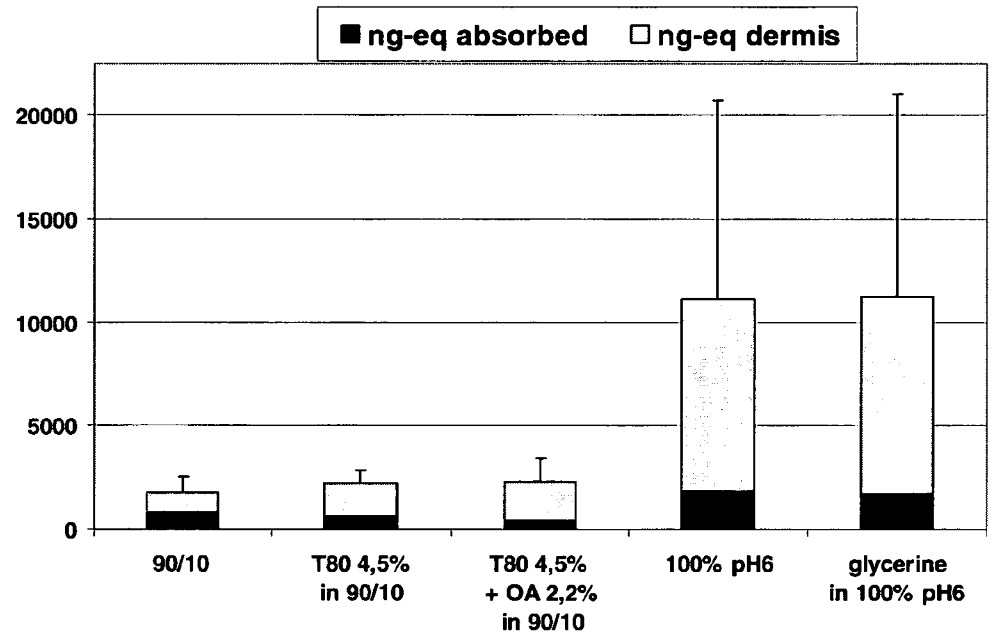

# FIGURE 13

EFFECT OF OLEIC ACID IN THE PRESENCE OF T80 AND OF GLYCERINE ON PERCUTANEOUS ABSORPTION OF RISEDRONATE IN BUFFERED HYDROALCOHOLIC SOLUTION (N=5 - 8; 2 SKINS)

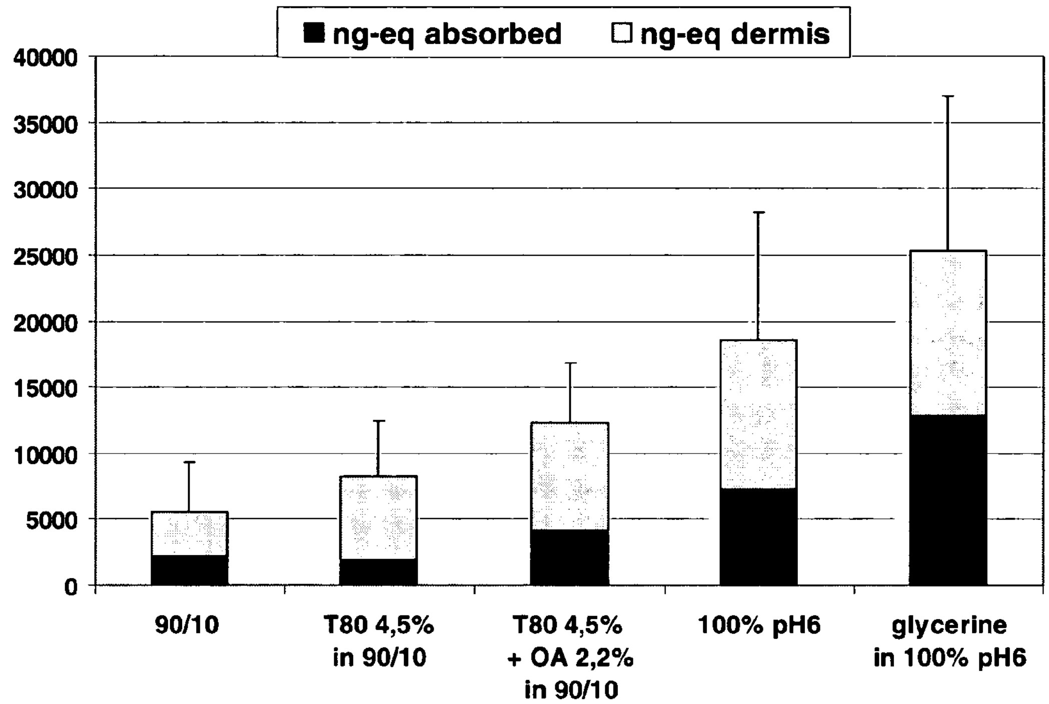

European Patent Office

# EUROPEAN SEARCH REPORT

Application Number
EP 06 29 1786

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 475 095 A (YAMANOUCHI PHARMA CO LTD [JP]; KYUKYU YAKUHIN KOGYO KK [JP]) 10 November 2004 (2004-11-10) * paragraphs [0011], [0068], [0071]; claims 1,2,8 * | 1-27 | INV. A61K9/00 |
| X | US 2005/074487 A1 (HSU TSUNG-MIN [US] ET AL) 7 April 2005 (2005-04-07) * paragraphs [0171] - [0173]; claims 1,20,37,52 * | 1-27 | |
| X | WO 2005/037157 A (DERMATRENDS INC [US]; HSU TSUNG-MIN [US]; JACOBSON ERIC [US]; LUO ERIC) 28 April 2005 (2005-04-28) * paragraphs [0038], [0045]; claims 1,20,36,39,40; example 1 * | 1-27 | |
| X | EP 0 600 834 A (CIBA GEIGY AG [CH]) 8 June 1994 (1994-06-08) * claims 1,12 * | 1-27 | |
| Y | US 2003/065294 A1 (PICKUP RAY L [US] ET AL) 3 April 2003 (2003-04-03) * claim 1 * | 11-20 | TECHNICAL FIELDS SEARCHED (IPC) |
| Y | US 6 406 451 B1 (ROWE JOHN [US]) 18 June 2002 (2002-06-18) * claim 1 * | 11-20 | A61K A61L A61M |

The present search report has been drawn up for all claims

| Place of search | Date of completion of the search | Examiner |
|---|---|---|
| Munich | 28 March 2007 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 06 29 1786

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2007

| Patent document cited in search report | | Publication date | Patent family member(s) | | | Publication date |
|---|---|---|---|---|---|---|
| EP 1475095 | A | 10-11-2004 | AU | 2003211963 | A1 | 04-09-2003 |
| | | | CN | 1633300 | A | 29-06-2005 |
| | | | WO | 03068241 | A1 | 21-08-2003 |
| | | | US | 2005106186 | A1 | 19-05-2005 |
| US 2005074487 | A1 | 07-04-2005 | NONE | | | |
| WO 2005037157 | A | 28-04-2005 | AU | 2004281766 | A1 | 28-04-2005 |
| | | | CA | 2542753 | A1 | 28-04-2005 |
| | | | CN | 1886105 | A | 27-12-2006 |
| | | | EP | 1682060 | A1 | 26-07-2006 |
| EP 0600834 | A | 08-06-1994 | NONE | | | |
| US 2003065294 | A1 | 03-04-2003 | CN | 1561242 | A | 05-01-2005 |
| | | | DE | 60212663 | T2 | 01-02-2007 |
| | | | EP | 1429835 | A1 | 23-06-2004 |
| | | | HK | 1062151 | A1 | 15-09-2006 |
| | | | JP | 2005503899 | T | 10-02-2005 |
| | | | WO | 03028797 | A1 | 10-04-2003 |
| | | | US | 2004087916 | A1 | 06-05-2004 |
| | | | US | 2004181196 | A1 | 16-09-2004 |
| US 6406451 | B1 | 18-06-2002 | CA | 2448366 | A1 | 28-11-2002 |
| | | | EP | 1389142 | A1 | 18-02-2004 |
| | | | WO | 02094367 | A1 | 28-11-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4705651 A
- EP 998292 A
- EP 1475095 A1
- US 6962691 B
- EP 1475095 A


### Non-patent literature cited in the description


- **J.H. LIN ; G. RUSSEL ; B.GERTZ.** Pharmacokinetics of alendronate: an overview. *Int J Clin Pract Suppl,* 1999, vol. 101, 18-26
- **J.H. LIN ; G. RUSSEL ; B.GERTZ.** Pharmacokinetics of alendronate : an overview. *Int J Clin Pract Suppl,* 1999, vol. 101, 18-26
- **J.H. LIN et al.** on the absorption of alendronate in rats. *J Pharm Sci,* vol. 83 (12), 1741-46